(19) Europäisches Patentamt
European Patent Office
Office européen des brevets

(11) Veröffentlichungsnummer: **0 010 673**
**B1**

(12) # EUROPÄISCHE PATENTSCHRIFT

(45) Veröffentlichungstag der Patentschrift: **27.01.82**

(51) Int. Cl.³: **C 07 C 103/34**, C 07 C 103/58, C 07 C 143/68, C 07 C 149/23, C 07 C 161/03, C 07 D 307/68, C 07 D 247/00, C 07 D 249/00, C 07 D 261/18, A 01 N 37/22, A 01 N 43/00

(21) Anmeldenummer: **79103965.4**

(22) Anmeldetag: **15.10.79**

(54) **Substituierte N-Propargyl-aniline, Verfahren zu ihrer Herstellung sowie ihre Verwendung als Fungizide.**

(30) Priorität: **31.10.78 DE 2847287**
**06.07.79 DE 2927461**

(43) Veröffentlichungstag der Anmeldung: **14.05.80 Patentblatt 80/10**

(45) Bekanntmachung des Hinweises auf die Patenterteilung: **27.01.82 Patentblatt 82/4**

(84) Benannte Vertragsstaaten: **AT BE CH DE FR GB IT NL SE**

(56) Entgegenhaltungen:
**DE-A1-2 349 256**
**DE-A1-2 513 732**
**DE-A1-2 702 102**
**EP-A1-0 005 591**
**US-A-3 535 377**
**US-A-4 001 325**
**US-A-4 124 376**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal-1 (DE)**
Erfinder: **Lunkenheimer, Winfried, Dr., Bismarkstrasse 29, D-5600 Wuppertal-1 (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 673 B1

BUNDESDRUCKEREI BERLIN

# Substituierte N-Propargyl-aniline, Verfahren zu ihrer Herstellung sowie ihre Verwendung als Fungizide (II)

Die vorliegende Erfindung betrifft neue substituierte N-Propargyl-aniline, mehrere Verfahren zu ihrer Herstellung und ihre Verwendung als Fungizide.

Es ist bereits bekanntgeworden, daß Halogenacetanilide, wie beispielsweise N-Chloracetyl-N-(2,6-dimethylphenyl)-alanin- bzw. -glycin-alkylester mit gutem Erfolg zur Bekämpfung von pilzlichen Pflanzenkrankheiten eingesetzt werden können (vgl. DE-OS 2 350 944 bzw. US-Patentschrift 3 780 090). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue substituierte N-Propargyl-aniline der allgemeinen Formel

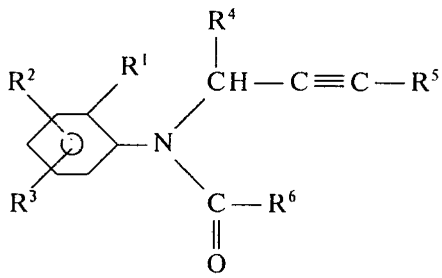

(I)

in welcher
$R^1$, $R^2$, $R^3$, $R^4$ und $R^5$ für Wasserstoff und
Alkyl mit 1 bis 4 Kohlenstoffatomen stehen, und darüber hinaus noch
$R^1$ zusätzlich für Halogen und
$R^5$ zusätzlich für gegebenenfalls durch Halogen, Alkyl mit 1 bis 4 Kohlenstoffatomen und Nitro substituiertes Phenyl und für Halogen steht,
$R^6$ für Tetrahydrofuryl, Thiophenyl, Tetrahydrothiophenyl, für gegebenenfalls durch Methyl oder Ethyl substituiertes Isoxazolyl, sowie für gegebenenfalls durch Cyano oder Thiocyano substituiertes Alkyl mit 1 bis 4 und Alkenyl und Alkinyl mit jeweils 2 bis 4 Kohlenstoffatomen steht, sowie für die Gruppierungen

$$-CH_2-Az, \quad -CH_2-OR^8, \quad -CH_2-SR^7, \quad -OR^7, \quad -CH_2-OSO_2R^8,$$

$-COOR^7$ und $-CH_2-O-$ 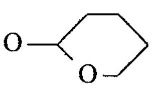

$R^7$ und $R^8$ für durch Halogen, Cyano und Thiocyano substituiertes Alkyl mit 1 bis 4 Kohlenstoffatomen und für gegebenenfalls durch die genannten Alkyl-Substituenten substituiertes Alkenyl und Alkinyl mit jeweils 2 bis 4 Kohlenstoffatomen und für Alkoxyalkyl mit 1 bis 4 Kohlenstoffatomen in jedem Alkylteil steht, und darüber hinaus noch
$R^7$ zusätzlich für unsubstituiertes Alkyl mit 1 bis 4 Kohlenstoffatomen stehen kann, und
Az für Pyrazol-1-yl, 1,2,4-Triazol-1-yl und Imidazol-1-yl steht,

gefunden. Sie weisen starke fungizide Eigenschaften auf.

Man erhält die substituierten N-Propargyl-aniline der Formel

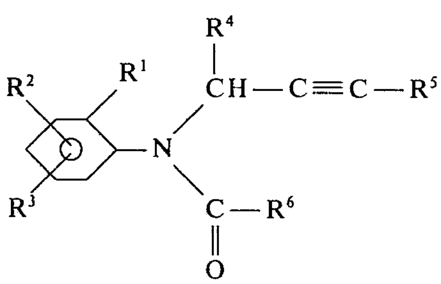

(I)

in welcher
$R^1$, $R^2$, $R^3$, $R^4$ und $R^5$ für Wasserstoff und
Alkyl mit 1 bis 4 Kohlenstoffatomen stehen, und darüber hinaus noch
$R^1$ zusätzlich für Halogen und
$R^5$ zusätzlich für gegebenenfalls durch Halogen, Alkyl mit 1 bis 4 Kohlenstoffatomen und Nitro substituiertes Phenyl und für Halogen steht,

$R^6$ für Tetrahydrofuryl, Thiophenyl, Tetrahydrothiophenyl, für gegebenenfalls durch Methyl oder Ethyl substituiertes Isoxazolyl, sowie für gegebenenfalls durch Cyano oder Thiocyano substituiertes Alkyl mit 1 bis 4 und Alkenyl und Alkinyl mit jeweils 2 bis 4 Kohlenstoffatomen steht, sowie für die Gruppierungen

$$-CH_2-Az, \quad -CH_2-OR^8, \quad -CH_2-SR^7, \quad -OR^7, \quad -CH_2-OSO_2R^8,$$

$-COOR^7$ und $-CH_2-O-$ 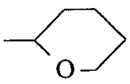

steht, wobei
$R^7$ und $R^8$ für durch Halogen, Cyano und Thiocyano substituiertes Alkyl mit 1 bis 4 Kohlenstoffatomen und für gegebenenfalls durch die genannten Alkyl-Substituenten substituiertes Alkenyl und Alkinyl mit jeweils 2 bis 4 Kohlenstoffatomen und für Alkoxyalkyl mit 1 bis 4 Kohlenstoffatomen in jedem Alkylteil steht, und darüber hinaus noch
$R^7$ zusätzlich für unsubstituiertes Alkyl mit 1 bis 4 Kohlenstoffatomen stehen kann, und
Az für Pyrazol-1-yl, 1,2,4-Triazol-1-yl und Imidazol-1-yl steht,
wenn man

a) N-Propargyl-aniline der Formel

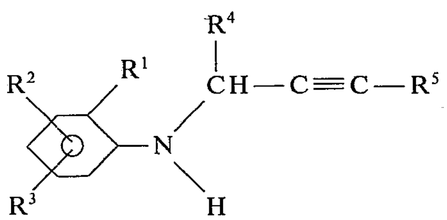

(II)

in welcher
$R^1$ bis $R^5$ die oben angegebene Bedeutung haben,
mit Säurechloriden oder -bromiden bzw. -anhydriden der Formeln

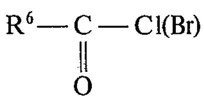

(IIIa)

bzw.

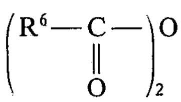

(IIIb)

in welchen
$R^6$ die oben angegebene Bedeutung hat, in Gegenwart eines Verdünnungsmittels und gegebenenfalls in Gegenwart eines Säurebindemittels umsetzt, oder

b) Anilide der Formel

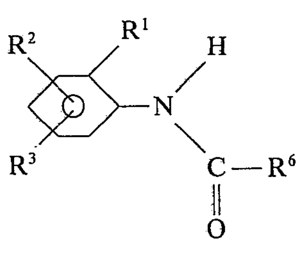

(IV)

in welcher
$R^1$ bis $R^3$ und $R^6$ die oben angegebene Bedeutung haben,
mit Propargylhalogeniden der Formel

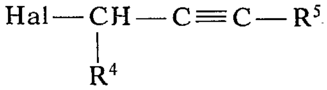

(V)

in welcher
$R^4$ und $R^5$ die oben angegebene Bedeutung haben, und
Hal für Chlor oder Brom steht,

in Gegenwart eines Säurebinders und gegebenenfalls in Gegenwart eines organischen Verdünnungsmittels oder in einem wäßrig-organischen Zweiphasensystem in Gegenwart eines Phasentransferkatalysators umsetzt; oder einzelne Verbindungen der Formel (I), indem man

c) Halogenacetanilide der Formel

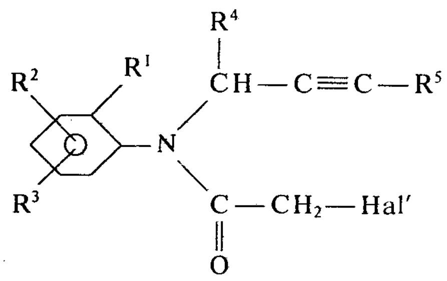

(VI)

in welcher
$R^1$ bis $R^5$ die oben angegebene Bedeutung haben, und
Hal' für Chlor, Brom oder Jod steht,
mit Verbindungen der Formel

$$B—X \qquad (VII)$$

in welcher
X für Az und die Gruppierung $-OR^8$ oder $-SR^7$ steht, und
B für Wasserstoff oder ein Alkalimetall steht, und
$R^7$ und $R^8$ die oben angegebene Bedeutung haben

gegebenenfalls in Gegenwart eines Verdünnungsmittels und gegebenenfalls in Gegenwart eines Säurebinders umsetzt, oder

d) Hydroxyacetanilide der Formel

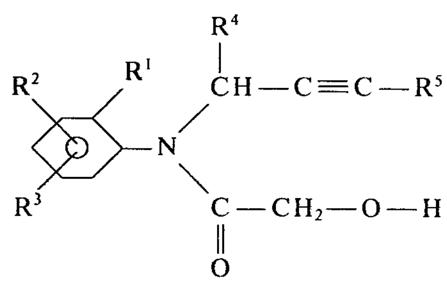

(VIII)

in welcher
$R^1$ bis $R^5$ die oben angegebene Bedeutung haben,

(1) gegebenenfalls nach Aktivierung mittels Alkalimetall mit Halogeniden der Formel

$$Hal'—R^9 \qquad (IX)$$

in welcher
Hal' die oben angegebene Bedeutung hat und
$R^9$ für den Rest $R^8$ sowie die Gruppe $-SO_2R^8$ steht, wobei
$R^8$ die oben angegebene Bedeutung hat,

in Gegenwart eines Verdünnungsmittels und gegebenenfalls in Gegenwart eines Säurebinders umsetzt, oder

(2) mit Dihydropyran der Formel

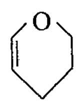

(X)

in Gegenwart eines Verdünnungsmittels und gegebenenfalls in Gegenwart eines Katalysators umsetzt, oder

e) erfindungsgemäße N-Propargyl-anilide der Formel

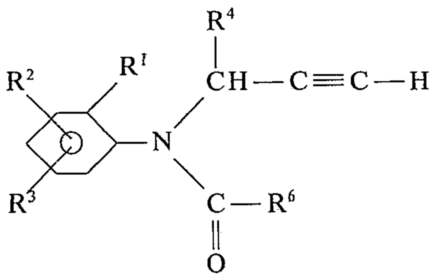

(Ia)

in welcher
$R^1$ bis $R^4$ und $R^6$ die oben angegebene Bedeutung haben,

in an sich bekannter Art und Weise mit Alkalihypohalogenid in Gegenwart eines Verdünnungsmittels umsetzt.

Die neuen substituierten N-Propargyl-aniline weisen starke fungizide Eigenschaften auf. Dabei zeigen überraschenderweise die erfindungsgemäßen Verbindungen eine erhebliche höhere Wirkung als die aus dem Stand der Technik bekanten N-Chloracetyl-N-(2,6-dimethylphenyl)-alanin- bzw. -glycin-alkylester.

Die erfindungsgemäßen substituierten N-Propargyl-aniline sind durch die Formel (I) allgemein definiert. In dieser Formel stehen $R^1$, $R^2$, $R^3$, $R^4$ und $R^5$ vorzugsweise für Wasserstoff und geradkettiges oder verzweigtes Alkyl mit 1 bis 4 Kohlenstoffatomen. $R^1$ steht außerdem vorzugsweise für Halogen, wie insbesondere Fluor, Chlor oder Brom. $R^5$ steht außerdem vorzugsweise für gegebenenfalls durch Halogen, Alkyl mit 1 bis 4 Kohlenstoffatomen und Nitro substituiertes Phenyl, sowie für Halogen.

$R^6$ steht vorzugsweise für Furyl, Tetrahydrofuryl, Thiophenyl, Tetrahydrothiophenyl, für gegebenenfalls durch Methyl oder Ethyl substituiertes Isoxazolyl sowie für gegebenenfalls durch Cyano oder Thiocyano substituiertes Alkyl mit 1 bis 4 und Alkenyl sowie Alkinyl mit jeweils 2 bis 4 Kohlenstoffatomen; ferner vorzugsweise für Dihalogenalkyl mit 1 bis 2 Kohlenstoffatomen, wobei als Halogenatome vorzugsweise Fluor und Chlor genannt seien, sowie für die Gruppierungen

$-CH_2-Az$, $-CH_2-OR^7$, $-CH_2-SR^7$, $-OR^7$, $-SR^7$, $-CH_2-OSO_2R^8$,

$-COOR^7$ und $-CH_2-O-$ 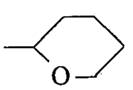 .

Az steht dabei vorzugsweise für Pyrazol-1-yl, 1,2,4-Triazol-1-yl und Imidazol-1-yl. $R^7$ steht vorzugsweise für gegebenenfalls durch Halogen, insbesondere Fluor, Chlor und Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil.

Ganz besonders bevorzugt sind diejenigen substituierten N-Propargyl-aniline der Formel (I), in denen $R^1$, $R^2$ und $R^3$ für Wasserstoff, Methyl, Ethyl, Isopropyl, sek.-Butyl und tert.-Butyl stehen; $R^1$ außerdem für Chlor oder Brom steht; $R^4$ für Wasserstoff, Methyl oder Ethyl steht; $R^5$ für Wasserstoff, Methyl, Ethyl, Phenyl, Brom, Chlor oder Jod steht; und $R^6$ für 2-Furyl, 2-Thienyl, 2-Tetrahydrofuryl, 5-Methylisoxazol-3-yl, Methoxymethyl, Ethoxymethyl, Allyloxymethyl, Propargyloxymethyl, Ethoxymethoxymethyl, Methylmercaptomethyl, Methoxy, Ethoxy, Methylmercapto, Methylsulfonyloxymethyl, Methoxycarbonyl, Ethoxycarbonyl, Dichlormethyl, Pyrazol-1-yl-methyl, Imidazol-1-yl-methyl, 1,2,4-Triazol-1-yl-methyl und Tetrahydropyran-2-yl-oxymethyl steht.

Im einzelnen seien außer den bei den Herstellungsbeispielen genannten Verbindungen die folgenden Verbindungen der allgemeinen Formel (I) genannt:

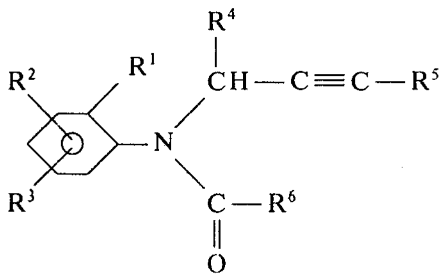

(I)

| $R^1$ | $R^2$ | $R^3$ | $R^4$ | $R^5$ | $R^6$ |
|---|---|---|---|---|---|
| $CH_3$ | 6-$CH_3$ | H | H | H | 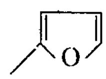 |
| $CH_3$ | 6-$CH_3$ | H | H | H | $-CH_2OCH_3$ |
| $CH_3$ | 6-$CH_3$ | H | H | H | $-CH_2-$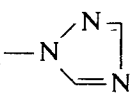 |
| $CH_3$ | 6-$CH_3$ | H | H | H | $-CH_2-$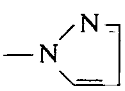 |
| $CH_3$ | 6-$CH_3$ | H | H | H | $-CH_2-$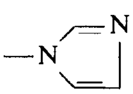 |
| $CH_3$ | 6-$CH_3$ | H | H | H | $-CH_2OC_2H_5$ |
| $CH_3$ | 6-$CH_3$ | H | H | H | $-CH_2-O-CH_2-OC_2H_5$ |
| $CH_3$ | 6-$CH_3$ | H | H | H | $-CH_2O-$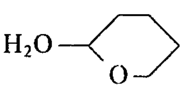 |
| $CH_3$ | 6-$CH_3$ | H | H | H | 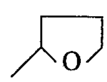 |
| $CH_3$ | 6-$CH_3$ | H | H | H | 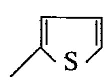 |
| $CH_3$ | 6-$CH_3$ | H | H | H | 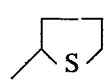 |
| $CH_3$ | 6-$CH_3$ | H | H | H | $-CH_2SCH_3$ |
| $CH_3$ | 6-$CH_3$ | H | H | H | $-CH_2SC_2H_5$ |
| $CH_3$ | 6-$CH_3$ | H | H | H | $-CHCl_2$ |
| $CH_3$ | 6-$CH_3$ | H | H | H | $-COOCH_3$ |
| $CH_3$ | 6-$CH_3$ | H | H | H | $-CH_2OSO_2CH_3$ |
| $CH_3$ | 6-$CH_3$ | H | H | $CH_3$ | 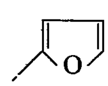 |

Fortsetzung

| $R^1$ | $R^2$ | $R^3$ | $R^4$ | $R^5$ | $R^6$ |
|---|---|---|---|---|---|
| $CH_3$ | 6-$CH_3$ | H | H | $CH_3$ | $-CH_2OCH_3$ |
| $CH_3$ | 6-$CH_3$ | H | H | $CH_3$ | $-CH_2-$ 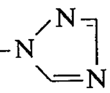 |
| $CH_3$ | 6-$CH_3$ | H | H | $CH_3$ | $-CH_2-$ 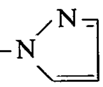 |
| $CH_3$ | 6-$CH_3$ | H | H | $CH_3$ | $-CH_2-$ 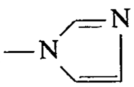 |
| $CH_3$ | 6-$CH_3$ | H | H | $CH_3$ | $-CH_2OC_2H_5$ |
| $CH_3$ | 6-$CH_3$ | H | H | $CH_3$ | $-CH_2OCH_2OC_2H_5$ |
| $CH_3$ | 6-$CH_3$ | H | H | $CH_3$ | $-CH_2O-$ 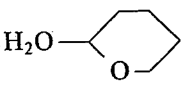 |
| $CH_3$ | 6-$CH_3$ | H | H | $CH_3$ | 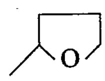 |
| $CH_3$ | 6-$CH_3$ | H | H | $CH_3$ | 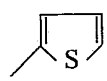 |
| $CH_3$ | 6-$CH_3$ | H | H | $CH_3$ | 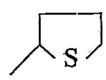 |
| $CH_3$ | 6-$CH_3$ | H | H | $CH_3$ | $-CH_2SCH_3$ |
| $CH_3$ | 6-$CH_3$ | H | H | $CH_3$ | $-CH_2SC_2H_5$ |
| $CH_3$ | 6-$CH_3$ | H | H | $CH_3$ | $-CHCl_2$ |
| $CH_3$ | 6-$CH_3$ | H | H | $CH_3$ | $-COOCH_3$ |
| $CH_3$ | 6-$CH_3$ | H | H | $CH_3$ | $-CH_2OSO_2CH_3$ |
| $CH_3$ | 6-$CH_3$ | H | $CH_3$ | H | 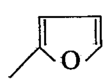 |
| $CH_3$ | 6-$CH_3$ | H | $CH_3$ | H | $-CH_2OCH_3$ |
| $CH_3$ | 6-$CH_3$ | H | $CH_3$ | H | $-CH_2-$ 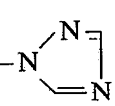 |
| $CH_3$ | 6-$CH_3$ | H | $CH_3$ | H | $-CH_2-$ 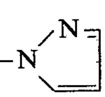 |
| $CH_3$ | 6-$CH_3$ | H | $CH_3$ | H | $-CH_2-$ 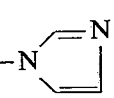 |

Fortsetzung

| $R^1$ | $R^2$ | $R^3$ | $R^4$ | $R^5$ | $R^6$ |
|---|---|---|---|---|---|
| $CH_3$ | 6-$CH_3$ | H | $CH_3$ | H | $-CH_2OC_2H_5$ |
| $CH_3$ | 6-$CH_3$ | H | $CH_3$ | H | $-CH_2-O-CH_2-OC_2H_5$ |
| $CH_3$ | 6-$CH_3$ | H | $CH_3$ | H | $-CH_2O-$ 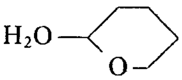 |
| $CH_3$ | 6-$CH_3$ | H | $CH_3$ | H | 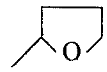 |
| $CH_3$ | 6-$CH_3$ | H | $CH_3$ | H | 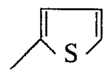 |
| $CH_3$ | 6-$CH_3$ | H | $CH_3$ | H | 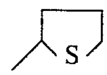 |
| $CH_3$ | 6-$CH_3$ | H | $CH_3$ | H | $-CH_2SCH_3$ |
| $CH_3$ | 6-$CH_3$ | H | $CH_3$ | H | $-CH_2SC_2H_5$ |
| $CH_3$ | 6-$CH_3$ | H | $CH_3$ | H | $-CHCl_2$ |
| $CH_3$ | 6-$CH_3$ | H | $CH_3$ | H | $-COOCH_3$ |
| $CH_3$ | 6-$CH_3$ | H | $CH_3$ | H | $-CH_2OSO_2CH_3$ |
| $CH_3$ | 6-$CH_3$ | H | H | Br | 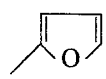 |
| $CH_3$ | 6-$CH_3$ | H | H | Br | $-CH_2OCH_3$ |
| $CH_3$ | 6-$CH_3$ | H | H | Br | $-CH_2-$ 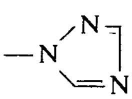 |
| $CH_3$ | 6-$CH_3$ | H | H | Br | $-CH_2-$ 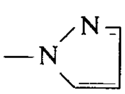 |
| $CH_3$ | 6-$CH_3$ | H | H | Br | $-CH_2-$ 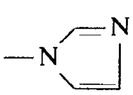 |
| $CH_3$ | 6-$CH_3$ | H | H | Br | $-CH_2OC_2H_5$ |
| $CH_3$ | 6-$CH_3$ | H | H | Br | $-CH_2-O-CH_2-OC_2H_5$ |
| $CH_3$ | 6-$CH_3$ | H | H | Br | $-CH_2O-$ 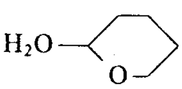 |
| $CH_3$ | 6-$CH_3$ | H | H | Br | 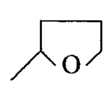 |
| $CH_3$ | 6-$CH_3$ | H | H | Br | 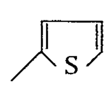 |

Fortsetzung

| $R^1$ | $R^2$ | $R^3$ | $R^4$ | $R^5$ | $R^6$ |
|---|---|---|---|---|---|
| $CH_3$ | 6-$CH_3$ | H | H | Br | 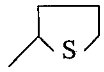 |
| $CH_3$ | 6-$CH_3$ | H | H | Br | $-CH_2SCH_3$ |
| $CH_3$ | 6-$CH_3$ | H | H | Br | $-CH_2SC_2H_5$ |
| $CH_3$ | 6-$CH_3$ | H | H | Br | $-CHCl_2$ |
| $CH_3$ | 6-$CH_3$ | H | H | Br | $-COOCH_3$ |
| $CH_3$ | 6-$CH_3$ | H | H | Br | $-CH_2OSO_2CH_3$ |
| $CH_3$ | 6-$CH_3$ | H | $CH_3$ | Br | 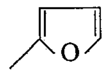 |
| $CH_3$ | 6-$CH_3$ | H | $CH_3$ | Br | $-CH_2OCH_3$ |
| $CH_3$ | 6-$CH_3$ | H | $CH_3$ | Br | $-CH_2-$ 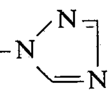 |
| $CH_3$ | 6-$CH_3$ | H | $CH_3$ | Br | $-CH_2-$ 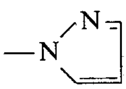 |
| $CH_3$ | 6-$CH_3$ | H | $CH_3$ | Br | $-CH_2-$ 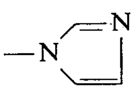 |
| $CH_3$ | 6-$CH_3$ | H | $CH_3$ | Br | $-CH_2OC_2H_5$ |
| $CH_3$ | 6-$CH_3$ | H | $CH_3$ | Br | $-CH_2-O-CH_2-OC_2H_5$ |
| $CH_3$ | 6-$CH_3$ | H | $CH_3$ | Br | $-CH_2O-$ 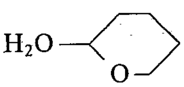 |
| $CH_3$ | 6-$CH_3$ | H | $CH_3$ | Br | 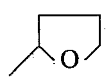 |
| $CH_3$ | 6-$CH_3$ | H | $CH_3$ | Br | 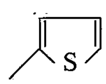 |
| $CH_3$ | 6-$CH_3$ | H | $CH_3$ | Br | 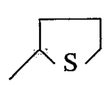 |
| $CH_3$ | 6-$CH_3$ | H | $CH_3$ | Br | $-CH_2SCH_3$ |
| $CH_3$ | 6-$CH_3$ | H | $CH_3$ | Br | $-CH_2SC_2H_5$ |
| $CH_3$ | 6-$CH_3$ | H | $CH_3$ | Br | $-CHCl_2$ |
| $CH_3$ | 6-$CH_3$ | H | $CH_3$ | Br | $-COOCH_3$ |
| $CH_3$ | 6-$CH_3$ | H | $CH_3$ | Br | $-CH_2OSO_2CH_3$ |

Fortsetzung

| $R^1$ | $R^2$ | $R^3$ | $R^4$ | $R^5$ | $R^6$ |
|---|---|---|---|---|---|
| $CH_3$ | 6-$CH_3$ | H | H | Cl | 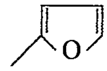 |
| $CH_3$ | 6-$CH_3$ | H | H | Cl | $-CH_2OCH_3$ |
| $CH_3$ | 6-$CH_3$ | H | H | Cl | $-CH_2-$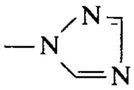 |
| $CH_3$ | 6-$CH_3$ | H | H | Cl | $-CH_2-$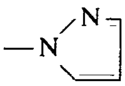 |
| $CH_3$ | 6-$CH_3$ | H | H | Cl | $-CH_2-$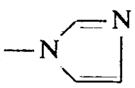 |
| $CH_3$ | 6-$CH_3$ | H | H | Cl | $-CH_2OC_2H_5$ |
| $CH_3$ | 6-$CH_3$ | H | H | Cl | $-CH_2O-CH_2-OC_2H_5$ |
| $CH_3$ | 6-$CH_3$ | H | H | Cl | $-CH_2O-$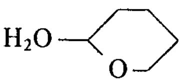 |
| $CH_3$ | 6-$CH_3$ | H | H | Cl | 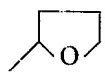 |
| $CH_3$ | 6-$CH_3$ | H | H | Cl | 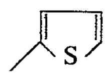 |
| $CH_3$ | 6-$CH_3$ | H | H | Cl | 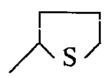 |
| $CH_3$ | 6-$CH_3$ | H | H | Cl | $-CH_2SCH_3$ |
| $CH_3$ | 6-$CH_3$ | H | H | Cl | $-CH_2-S-C_2H_5$ |
| $CH_3$ | 6-$CH_3$ | H | H | Cl | $-CHCl_2$ |
| $CH_3$ | 6-$CH_3$ | H | H | Cl | $-COOCH_3$ |
| $CH_3$ | 6-$CH_3$ | H | H | Cl | $-CH_2OSO_2CH_3$ |
| $CH_3$ | 6-$CH_3$ | H | H | J | 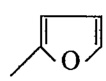 |
| $CH_3$ | 6-$CH_3$ | H | H | J | $-CH_2OCH_3$ |
| $CH_3$ | 6-$CH_3$ | H | H | J | $-CH_2-$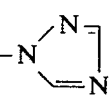 |
| $CH_3$ | 6-$CH_3$ | H | H | J | $-CH_2-$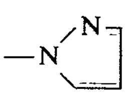 |

Fortsetzung

| $R^1$ | $R^2$ | $R^3$ | $R^4$ | $R^5$ | $R^6$ |
|---|---|---|---|---|---|
| $CH_3$ | 6-$CH_3$ | H | H | J | —$CH_2$— 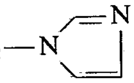 |
| $CH_3$ | 6-$CH_3$ | H | H | J | —$CH_2OC_2H_5$ |
| $CH_3$ | 6-$CH_3$ | H | H | J | —$CH_2O$—$CH_2$—$OC_2H_5$ |
| $CH_3$ | 6-$CH_3$ | H | H | J | —$CH_2O$— 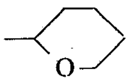 |
| $CH_3$ | 6-$CH_3$ | H | H | J | 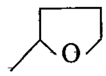 |
| $CH_3$ | 6-$CH_3$ | H | H | J | 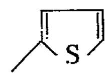 |
| $CH_3$ | 6-$CH_3$ | H | H | J | 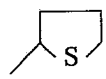 |
| $CH_3$ | 6-$CH_3$ | H | H | J | —$CH_2SCH_3$ |
| $CH_3$ | 6-$CH_3$ | H | H | J | —$CH_2$—S—$C_2H_5$ |
| $CH_3$ | 6-$CH_3$ | H | H | J | —$COOCH_3$ |
| $CH_3$ | 6-$CH_3$ | H | H | J | —$CH_2OSO_2CH_3$ |
| $CH_3$ | 6-$C_2H_5$ | H | H | H | 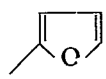 |
| $CH_3$ | 6-$C_2H_5$ | H | H | H | —$CH_2OCH_3$ |
| $CH_3$ | 6-$C_2H_5$ | H | H | H | —$CH_2$— 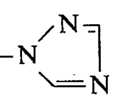 |
| $CH_3$ | 6-$C_2H_5$ | H | H | H | —$CH_2$— 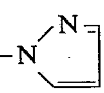 |
| $CH_3$ | 6-$C_2H_5$ | H | H | H | —$CH_2OC_2H_5$ |
| $CH_3$ | 6-$C_2H_5$ | H | H | H | —$CH_2OSO_2CH_3$ |
| $CH_3$ | 6-$C_2H_5$ | H | H | H | —$CHCl_2$ |
| $CH_3$ | 6-$C_2H_5$ | H | H | H | —$COOCH_3$ |
| $CH_3$ | 6-$C_2H_5$ | H | H | H | 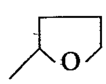 |
| $CH_3$ | 6-$C_2H_5$ | H | H | H | —$CH_2SCH_3$ |
| $CH_3$ | 6-$C_2H_5$ | H | H | H | —$CH_2O$ 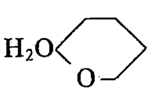 |

Fortsetzung

| $R^1$ | $R^2$ | $R^3$ | $R^4$ | $R^5$ | $R^6$ |
|---|---|---|---|---|---|
| $CH_3$ | 6-$C_2H_5$ | H | $CH_3$ | H | 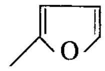 |
| $CH_3$ | 6-$C_2H_5$ | H | $CH_3$ | H | $-CH_2OCH_3$ |
| $CH_3$ | 6-$C_2H_5$ | H | $CH_3$ | H | $-CH_2-$ 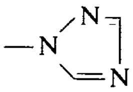 |
| $CH_3$ | 6-$C_2H_5$ | H | $CH_3$ | H | $-CH_2-$ 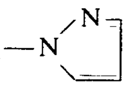 |
| $CH_3$ | 6-$C_2H_5$ | H | $CH_3$ | H | $-CH_2OC_2H_5$ |
| $CH_3$ | 6-$C_2H_5$ | H | $CH_3$ | H | $-CH_2OSO_2CH_3$ |
| $CH_3$ | 6-$C_2H_5$ | H | $CH_3$ | H | $-CHCl_2$ |
| $CH_3$ | 6-$C_2H_5$ | H | $CH_3$ | H | $-COOCH_3$ |
| $CH_3$ | 6-$C_2H_5$ | H | $CH_3$ | H | 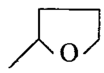 |
| $CH_3$ | 6-$C_2H_5$ | H | $CH_3$ | H | $-CH_2SCH_3$ |
| $CH_3$ | 6-$C_2H_5$ | H | $CH_3$ | H | $-CH_2O$ 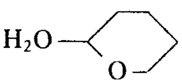 |
| $CH_3$ | 6-$C_2H_5$ | H | H | $CH_3$ | 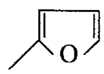 |
| $CH_3$ | 6-$C_2H_5$ | H | H | $CH_3$ | $-CH_2OCH_3$ |
| $CH_3$ | 6-$C_2H_5$ | H | H | $CH_3$ | $-CH_2-$ 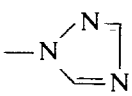 |
| $CH_3$ | 6-$C_2H_5$ | H | H | $CH_3$ | $-CH_2-$ 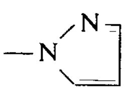 |
| $CH_3$ | 6-$C_2H_5$ | H | H | $CH_3$ | $-CH_2OC_2H_5$ |
| $CH_3$ | 6-$C_2H_5$ | H | H | $CH_3$ | $-CH_2OSO_2CH_3$ |
| $CH_3$ | 6-$C_2H_5$ | H | H | $CH_3$ | $-CHCl_2$ |
| $CH_3$ | 6-$C_2H_5$ | H | H | $CH_3$ | $-COOCH_3$ |
| $CH_3$ | 6-$C_2H_5$ | H | H | $CH_3$ | 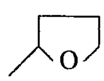 |
| $CH_3$ | 6-$C_2H_5$ | H | H | $CH_3$ | $-CH_2SCH_3$ |

Fortsetzung

| $R^1$ | $R^2$ | $R^3$ | $R^4$ | $R^5$ | $R^6$ |
|---|---|---|---|---|---|
| $CH_3$ | 6-$C_2H_5$ | H | H | $CH_3$ | 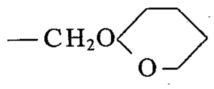 |
| $CH_3$ | 6-$C_2H_5$ | H | H | Br | 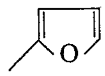 |
| $CH_3$ | 6-$C_2H_5$ | H | H | Br | $-CH_2OCH_3$ |
| $CH_3$ | 6-$C_2H_5$ | H | H | Br | 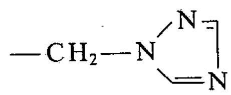 |
| $CH_3$ | 6-$C_2H_5$ | H | H | Br | 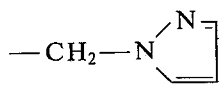 |
| $CH_3$ | 6-$C_2H_5$ | H | H | Br | $-CH_2OC_2H_5$ |
| $CH_3$ | 6-$C_2H_5$ | H | H | Br | $-CH_2OSO_2CH_3$ |
| $CH_3$ | 6-$C_2H_5$ | H | H | Br | $-CHCl_2$ |
| $CH_3$ | 6-$C_2H_5$ | H | H | Br | $-COOCH_3$ |
| $CH_3$ | 6-$C_2H_5$ | H | H | Br | 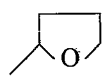 |
| $CH_3$ | 6-$C_2H_5$ | H | H | Br | $-CH_2SCH_3$ |
| $CH_3$ | 6-$C_2H_5$ | H | H | Br | 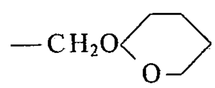 |
| $CH_3$ | 6-$C_2H_5$ | H | $CH_3$ | Br | 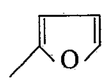 |
| $CH_3$ | 6-$C_2H_5$ | H | $CH_3$ | Br | $-CH_2OCH_3$ |
| $CH_3$ | 6-$C_2H_5$ | H | $CH_3$ | Br | 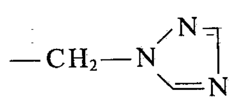 |
| $CH_3$ | 6-$C_2H_5$ | H | $CH_3$ | Br | 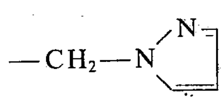 |
| $CH_3$ | 6-$C_2H_5$ | H | $CH_3$ | Br | $-CH_2OC_2H_5$ |
| $CH_3$ | 6-$C_2H_5$ | H | $CH_3$ | Br | $-CH_2OSO_2CH_3$ |
| $CH_3$ | 6-$C_2H_5$ | H | $CH_3$ | Br | $-CHCl_2$ |
| $CH_3$ | 6-$C_2H_5$ | H | $CH_3$ | Br | $-COOCH_3$ |
| $CH_3$ | 6-$C_2H_5$ | H | $CH_3$ | Br | 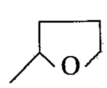 |

Fortsetzung

| $R^1$ | $R^2$ | $R^3$ | $R^4$ | $R^5$ | $R^6$ |
|---|---|---|---|---|---|
| $CH_3$ | 6-$C_2H_5$ | H | $CH_3$ | Br | $-CH_2SCH_3$ |
| $CH_3$ | 6-$C_2H_5$ | H | $CH_3$ | Br | $-CH_2O-$ 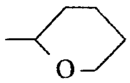 |
| $CH_3$ | 6-$C_2H_5$ | H | H | Cl | 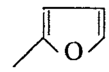 |
| $CH_3$ | 6-$C_2H_5$ | H | H | Cl | $-CH_2OCH_3$ |
| $CH_3$ | 6-$C_2H_5$ | H | H | Cl | $-CH_2-$ 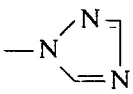 |
| $CH_3$ | 6-$C_2H_5$ | H | H | Cl | $-CH_2-$ 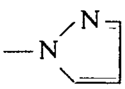 |
| $CH_3$ | 6-$C_2H_5$ | H | H | Cl | $-CH_2OC_2H_5$ |
| $CH_3$ | 6-$C_2H_5$ | H | H | Cl | $-CH_2OSO_2CH_3$ |
| $CH_3$ | 6-$C_2H_5$ | H | H | Cl | $-CHCl_2$ |
| $CH_3$ | 6-$C_2H_5$ | H | H | Cl | $-COOCH_3$ |
| $CH_3$ | 6-$C_2H_5$ | H | H | Cl | 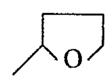 |
| $CH_3$ | 6-$C_2H_5$ | H | H | Cl | $-CH_2SCH_3$ |
| $CH_3$ | 6-$C_2H_5$ | H | H | Cl | $-CH_2O$ 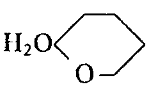 |
| $CH_3$ | 6-$C_2H_5$ | H | H | J | 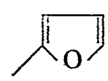 |
| $CH_3$ | 6-$C_2H_5$ | H | H | J | $-CH_2OCH_3$ |
| $CH_3$ | 6-$C_2H_5$ | H | H | J | $-CH_2-$ 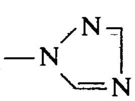 |
| $CH_3$ | 6-$C_2H_5$ | H | H | J | $-CH_2-$ 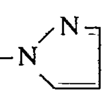 |
| $CH_3$ | 6-$C_2H_5$ | H | H | J | $-CH_2OC_2H_5$ |
| $CH_3$ | 6-$C_2H_5$ | H | H | J | $-CH_2OSO_2CH_3$ |
| $CH_3$ | 6-$C_2H_5$ | H | H | J | $-CHCl_2$ |
| $CH_3$ | 6-$C_2H_5$ | H | H | J | $-COOCH_3$ |

Fortsetzung

| $R^1$ | $R^2$ | $R^3$ | $R^4$ | $R^5$ | $R^6$ |
|---|---|---|---|---|---|
| $CH_3$ | 6-$C_2H_5$ | H | H | J | 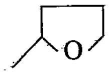 |
| $CH_3$ | 6-$C_2H_5$ | H | H | J | $-CH_2SCH_3$ |
| $CH_3$ | 6-$C_2H_5$ | H | H | J | $-CH_2O$ 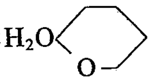 |
| $C_2H_5$ | 6-$C_2H_5$ | H | H | H | 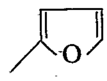 |
| $C_2H_5$ | 6-$C_2H_5$ | H | H | H | $-CH_2OCH_3$ |
| $C_2H_5$ | 6-$C_2H_5$ | H | H | H | $-CH_2-$ 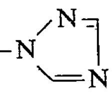 |
| $C_2H_5$ | 6-$C_2H_5$ | H | H | H | $-CH_2-$ 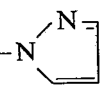 |
| $C_2H_5$ | 6-$C_2H_5$ | H | H | H | $-CH_2OC_2H_5$ |
| $C_2H_5$ | 6-$C_2H_5$ | H | H | H | $-CH_2OSO_2CH_3$ |
| $C_2H_5$ | 6-$C_2H_5$ | H | H | H | $-CHCl_2$ |
| $C_2H_5$ | 6-$C_2H_5$ | H | H | H | $-COOCH_3$ |
| $C_2H_5$ | 6-$C_2H_5$ | H | H | H | 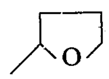 |
| $C_2H_5$ | 6-$C_2H_5$ | H | H | H | $-CH_2SCH_3$ |
| $C_2H_5$ | 6-$C_2H_5$ | H | H | H | $-CH_2O-$ 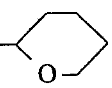 |
| $C_2H_5$ | 6-$C_2H_5$ | H | $CH_3$ | H | 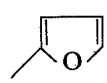 |
| $C_2H_5$ | 6-$C_2H_5$ | H | $CH_3$ | H | $-CH_2OCH_3$ |
| $C_2H_5$ | 6-$C_2H_5$ | H | $CH_3$ | H | $-CH_2-$ 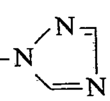 |
| $C_2H_5$ | 6-$C_2H_5$ | H | $CH_3$ | H | $-CH_2-$ 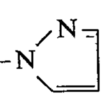 |
| $C_2H_5$ | 6-$C_2H_5$ | H | $CH_3$ | H | $-CH_2OC_2H_5$ |
| $C_2H_5$ | 6-$C_2H_5$ | H | $CH_3$ | H | $-CH_2OSO_2CH_3$ |
| $C_2H_5$ | 6-$C_2H_5$ | H | $CH_3$ | H | $-CHCl_2$ |

Fortsetzung

| $R^1$ | $R^2$ | $R^3$ | $R^4$ | $R^5$ | $R^6$ |
|---|---|---|---|---|---|
| $C_2H_5$ | 6-$C_2H_5$ | H | $CH_3$ | H | $-COOCH_3$ |
| $C_2H_5$ | 6-$C_2H_5$ | H | $CH_3$ | H | 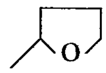 |
| $C_2H_5$ | 6-$C_2H_5$ | H | $CH_3$ | H | $-CH_2SCH_3$ |
| $C_2H_5$ | 6-$C_2H_5$ | H | $CH_3$ | H | $-CH_2O-$ 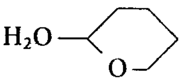 |
| $C_2H_5$ | 6-$C_2H_5$ | H | H | $CH_3$ | 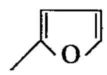 |
| $C_2H_5$ | 6-$C_2H_5$ | H | H | $CH_3$ | $-CH_2OCH_3$ |
| $C_2H_5$ | 6-$C_2H_5$ | H | H | $CH_3$ | $-CH_2-$ 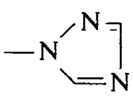 |
| $C_2H_5$ | 6-$C_2H_5$ | H | H | $CH_3$ | $-CH_2-$ 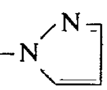 |
| $C_2H_5$ | 6-$C_2H_5$ | H | H | $CH_3$ | $-CH_2OC_2H_5$ |
| $C_2H_5$ | 6-$C_2H_5$ | H | H | $CH_3$ | $-CH_2OSO_2CH_3$ |
| $C_2H_5$ | 6-$C_2H_5$ | H | H | $CH_3$ | $-CHCl_2$ |
| $C_2H_5$ | 6-$C_2H_5$ | H | H | $CH_3$ | $-COOCH_3$ |
| $C_2H_5$ | 6-$C_2H_5$ | H | H | $CH_3$ | 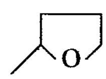 |
| $C_2H_5$ | 6-$C_2H_5$ | H | H | $CH_3$ | $-CH_2SCH_3$ |
| $C_2H_5$ | 6-$C_2H_5$ | H | H | $CH_3$ | $-CH_2O-$ 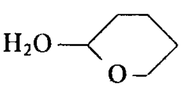 |
| $C_2H_5$ | 6-$C_2H_5$ | H | H | Br | 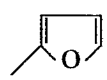 |
| $C_2H_5$ | 6-$C_2H_5$ | H | H | Br | $-CH_2OCH_3$ |
| $C_2H_5$ | 6-$C_2H_5$ | H | H | Br | $-CH_2-$ 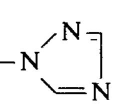 |
| $C_2H_5$ | 6-$C_2H_5$ | H | H | Br | $-CH_2-$ 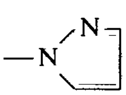 |
| $C_2H_5$ | 6-$C_2H_5$ | H | H | Br | $-CH_2OC_2H_5$ |
| $C_2H_5$ | 6-$C_2H_5$ | H | H | Br | $-CH_2OSO_2CH_3$ |

Fortsetzung

| $R^1$ | $R^2$ | $R^3$ | $R^4$ | $R^5$ | $R^6$ |
|---|---|---|---|---|---|
| $C_2H_5$ | 6-$C_2H_5$ | H | H | Br | $-CHCl_2$ |
| $C_2H_5$ | 6-$C_2H_5$ | H | H | Br | $-COOCH_3$ |
| $C_2H_5$ | 6-$C_2H_5$ | H | H | Br | 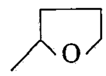 |
| $C_2H_5$ | 6-$C_2H_5$ | H | H | Br | $-CH_2SCH_3$ |
| $C_2H_5$ | 6-$C_2H_5$ | H | H | Br | $-CH_2O-$ 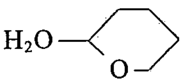 |
| $C_2H_5$ | 6-$C_2H_5$ | H | $CH_3$ | Br | 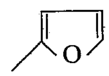 |
| $C_2H_5$ | 6-$C_2H_5$ | H | $CH_3$ | Br | $-CH_2OCH_3$ |
| $C_2H_5$ | 6-$C_2H_5$ | H | $CH_3$ | Br | $-CH_2-$ 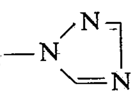 |
| $C_2H_5$ | 6-$C_2H_5$ | H | $CH_3$ | Br | $-CH_2-$ 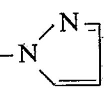 |
| $C_2H_5$ | 6-$C_2H_5$ | H | $CH_3$ | Br | $-CH_2OC_2H_5$ |
| $C_2H_5$ | 6-$C_2H_5$ | H | $CH_3$ | Br | $-CH_2OSO_2CH_3$ |
| $C_2H_5$ | 6-$C_2H_5$ | H | $CH_3$ | Br | $-CHCl_2$ |
| $C_2H_5$ | 6-$C_2H_5$ | H | $CH_3$ | Br | $-COOCH_3$ |
| $C_2H_5$ | 6-$C_2H_5$ | H | $CH_3$ | Br | 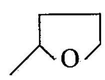 |
| $C_2H_5$ | 6-$C_2H_5$ | H | $CH_3$ | Br | $-CH_2SCH_3$ |
| $C_2H_5$ | 6-$C_2H_5$ | H | $CH_3$ | Br | $-CH_2O-$ 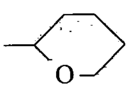 |
| $C_2H_5$ | 6-$C_2H_5$ | H | H | Cl | 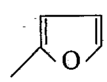 |
| $C_2H_5$ | 6-$C_2H_5$ | H | H | Cl | $-CH_2OCH_3$ |
| $C_2H_5$ | 6-$C_2H_5$ | H | H | Cl | $-CH_2-$ 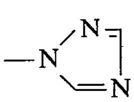 |
| $C_2H_5$ | 6-$C_2H_5$ | H | H | Cl | $-CH_2-$ 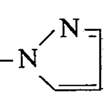 |
| $C_2H_5$ | 6-$C_2H_5$ | H | H | Cl | $-CH_2OC_2H_5$ |

Fortsetzung

| $R^1$ | $R^2$ | $R^3$ | $R^4$ | $R^5$ | $R^6$ |
|---|---|---|---|---|---|
| $C_2H_5$ | 6-$C_2H_5$ | H | H | Cl | $-CH_2OSO_2CH_3$ |
| $C_2H_5$ | 6-$C_2H_5$ | H | H | Cl | $-CHCl_2$ |
| $C_2H_5$ | 6-$C_2H_5$ | H | H | Cl | $-COOCH_3$ |
| $C_2H_5$ | 6-$C_2H_5$ | H | H | Cl | 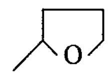 |
| $C_2H_5$ | 6-$C_2H_5$ | H | H | Cl | $-CH_2SCH_3$ |
| $C_2H_5$ | 6-$C_2H_5$ | H | H | Cl | $-CH_2O$ 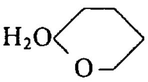 |
| $C_2H_5$ | 6-$C_2H_5$ | H | H | J | 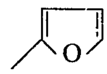 |
| $C_2H_5$ | 6-$C_2H_5$ | H | H | J | $-CH_2OCH_3$ |
| $C_2H_5$ | 6-$C_2H_5$ | H | H | J | $-CH_2-$ 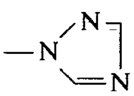 |
| $C_2H_5$ | 6-$C_2H_5$ | H | H | J | $-CH_2-$ 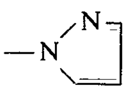 |
| $C_2H_5$ | 6-$C_2H_5$ | H | H | J | $-CH_2OC_2H_5$ |
| $C_2H_5$ | 6-$C_2H_5$ | H | H | J | $-CH_2OSO_2CH_3$ |
| $C_2H_5$ | 6-$C_2H_5$ | H | H | J | $-CHCl_2$ |
| $C_2H_5$ | 6-$C_2H_5$ | H | H | J | $-COOCH_3$ |
| $C_2H_5$ | 6-$C_2H_5$ | H | H | J | 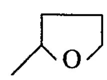 |
| $C_2H_5$ | 6-$C_2H_5$ | H | H | J | $-CH_2SCH_3$ |
| $C_2H_5$ | 6-$C_2H_5$ | H | H | J | $-CH_2O$ 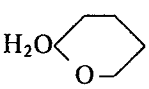 |

Verwendet man beispielsweise 2,6-Dimethyl-N-propargyl-anilin und Furan-2-carbonsäurechlorid als Ausgangsstoffe, so kann der Reaktionsablauf durch das folgende Formelschema wiedergegeben werden (Verfahren a):

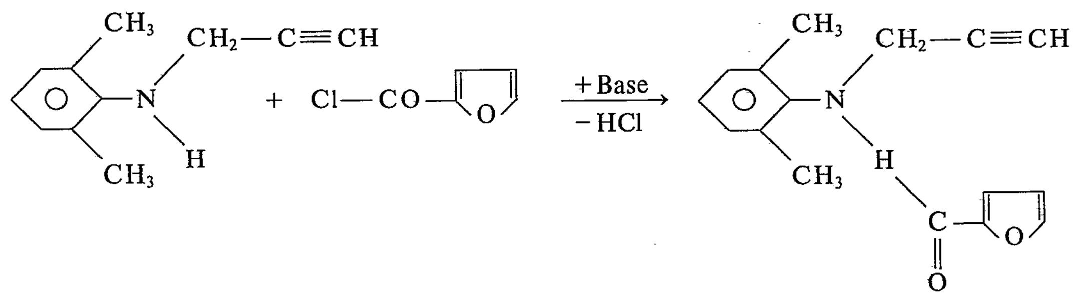


Verwendet man beispielsweise 2,6-Dimethyl-N-(2-furoyl)-anilin und Propargylbromid als Ausgangsstoffe, so kann der Reaktionsablauf durch das folgende Formelschema wiedergegeben werden (Verfahren b):

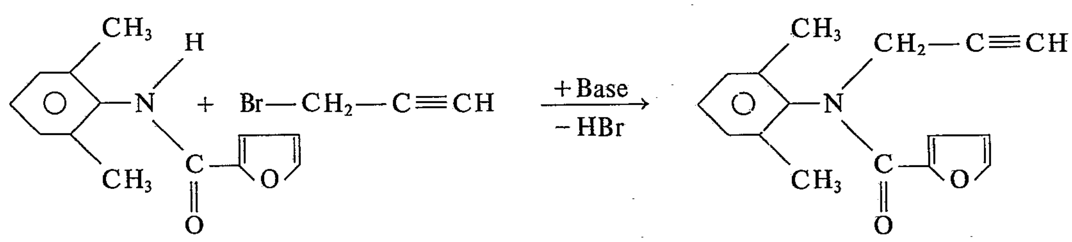


Verwendet man beispielsweise 2,6-Dimethyl-N-propargyl-chloracetanilid und Pyrazol als Ausgangsstoffe, so kann der Reaktionsablauf durch das folgende Formelschema wiedergegeben werden (Verfahren c):

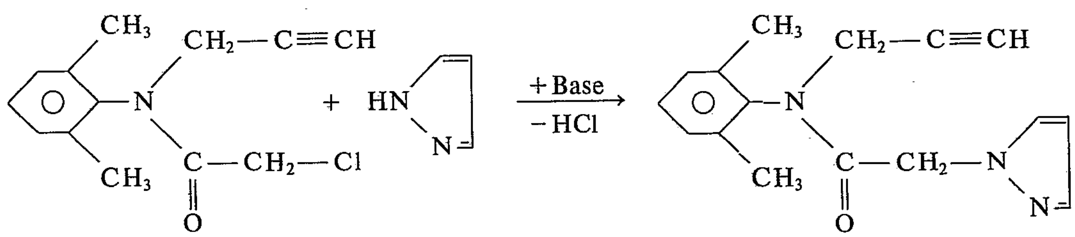


Verwendet man beispielsweise 2,6-Dimethyl-N-propargyl-hydroxyacetanilid und Ethoxymethylchlorid als Ausgangsstoffe, so kann der Reaktionsablauf durch das folgende Formelschema wiedergegeben werden (Verfahren d/1):

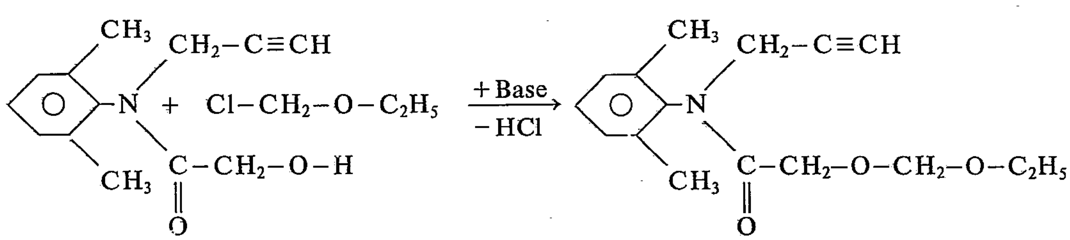


Verwendet man beispielsweise 2,6-Dimethyl-N-propargyl-hydroxyacetanilid und 3,4-Dihydro-2H-pyran als Ausgangsstoffe, so kann der Reaktionsablauf durch das folgende Formelschema wiedergegeben werden (Verfahren d/2):

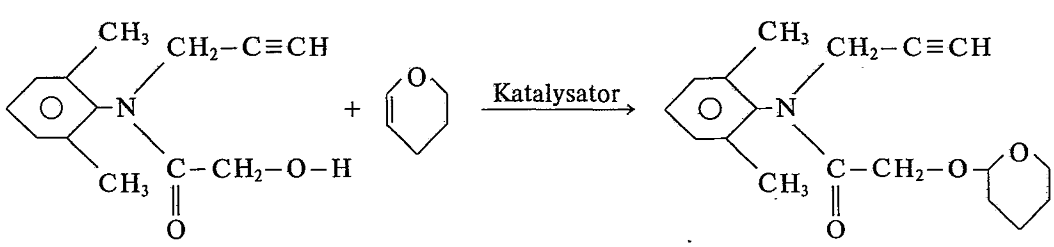

Verwendet man beispielsweise 2,6-Dimethyl-N-(2-furoyl)-N-propargyl-anilin und Kaliumhypobromit als Ausgangsstoffe, so kann der Reaktionsablauf durch das folgende Formelschema wiedergegeben werden (Verfahren e):

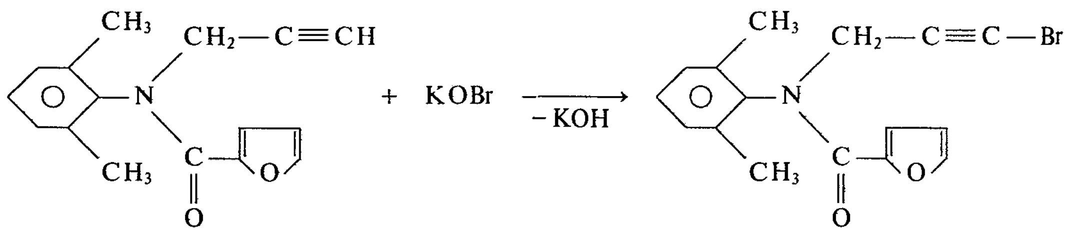


Die bei der Durchführung des erfindungsgemäßen Verfahrens (a) als Ausgangsstoffe benötigten N-Propargyl-aniline sind durch die Formel (II) allgemein definiert. In dieser Formel stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Propargyl-aniline der Formel (II) sind teilweise bekannt (vergleiche die US-Patentschriften 3 535 377 und 4 001 325), bzw. können sie nach bekannten Verfahren erhalten werden, indem man z. B. entsprechende Aniline mit Propargylhalogeniden der Formel (V) oder den entsprechenden Propargylsulfonaten, wie beispielsweise Mesylaten oder Tosylaten, in Gegenwart eines Säurebinders, wie z. B. Natrium- oder Kaliumcarbonat, gegebenenfalls in Gegenwart eines inerten organischen Lösungsmittels, wie beispielsweise Ethanol, bei Temperaturen zwischen 20 und 150°C umsetzt, wobei vorzugsweise auch ein Überschuß an Anilin eingesetzt werden kann.

N-Propargyl-aniline der Formel (II), bei denen $R^4$ für Methyl steht, können auch durch Umsetzung der entsprechenden Aniline mit Acetylen in Gegenwart von Kupferacetylid unter Druck erhalten werden (vergleiche hierzu Liebigs Ann. Chem. 596, 1 [1955]).

Als Beispiele für die Ausgangsstoffe der Formel (II) seien genannt:

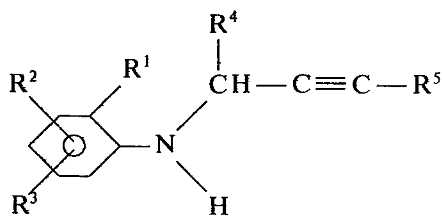

(II)

| $R^1$ | $R^2$ | $R^3$ | $R^4$ | $R^5$ |
|---|---|---|---|---|
| $CH_3$ | 6-$CH_3$ | H | H | H |
| $C_2H_5$ | 6-$C_2H_5$ | H | H | H |
| $C_2H_5$ | 6-$CH_3$ | H | H | H |
| $C(CH_3)_3$ | H | H | H | H |
| $CH_3$ | 3-$CH_3$ | H | H | H |
| $CH_3$ | 5-$CH_3$ | H | H | H |
| Cl | 6-$CH_3$ | H | H | H |
| Cl | 6-$C(CH_3)_3$ | H | H | H |
| $CH_3$ | 3-$CH_3$ | 6-$CH_3$ | H | H |
| $CH_3$ | 6-$CH_3$ | H | $CH_3$ | H |
| $C_2H_5$ | 6-$C_2H_5$ | H | $CH_3$ | H |
| $C_2H_5$ | 6-$CH_3$ | H | $CH_3$ | H |
| $CH_3$ | 6-$CH_3$ | H | $C_2H_5$ | H |
| $C_2H_5$ | 6-$C_2H_5$ | H | $C_2H_5$ | H |
| $C_2H_5$ | 6-$CH_3$ | H | $C_2H_5$ | H |
| $CH_3$ | 6-$CH_3$ | H | H | $CH_3$ |
| $C_2H_5$ | 6-$C_2H_5$ | H | H | $CH_3$ |
| $C_2H_5$ | 6-$CH_3$ | H | H | $CH_3$ |
| $CH_3$ | 6-$CH_3$ | H | H | 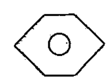 |
| $C_2H_5$ | 6-$C_2H_5$ | H | H | 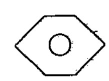 |
| $C_2H_5$ | 6-$CH_3$ | H | H | 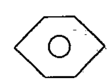 |
| $CH_3$ | 6-$CH_3$ | H | $CH_3$ | $CH_3$ |
| $C_2H_5$ | 6-$C_2H_5$ | H | $CH_3$ | $CH_3$ |
| $C_2H_5$ | 6-$CH_3$ | H | $CH_3$ | $CH_3$ |

Die außerdem für das erfindungsgemäße Verfahren (a) als Ausgangsstoffe zu verwendenden Säurechloride oder -bromide bzw. -anhydride sind durch die Formeln (IIIa) und (IIIb) allgemein definiert. In diesen Formeln steht $R^6$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Säurechloride oder -bromide bzw. -anhydride der Formeln (IIIa) und (IIIb) sind allgemein bekannte Verbindungen der organischen Chemie.

Die bei der Durchführung des erfindungsgemäßen Verfahrens (b) als Ausgangsstoffe benötigten Anilide sind durch die Formel (IV) allgemein definiert. In dieser Formel stehen $R^1$, $R^2$, $R^3$ und $R^6$ vorzugsweise für diejenigen Reste, die bereits im Zusammenhang mit der Beschreibung der erfindungsgemäßen Stoffe der Formel (I) vorzugsweise für diese Substituenten genannt wurden.

Die Anilide der Formel (IV) können in allgemein bekannter Art und Weise erhalten werden, indem man entsprechende Aniline mit einem Säurechlorid oder -bromid bzw. -anhydrid der Formeln (IIIa) und (IIIb) gemäß den Bedingungen des Verfahrens (a) in Gegenwart eines inerten organischen Lösungsmittels wie beispielsweise Toluol oder Methylenchlorid, gegebenenfalls in Gegenwart eines Säurebindemittels, wie z. B. Kaliumcarbonat oder Triethylamin, oder in Gegenwart eines Katalysators, wie z. B. Dimethylformamid, bei Temperaturen zwischen 0 und 100°C umsetzt (vergleiche auch die Herstellungsbeispiele).

Als Beispiele für die Ausgangsstoffe der Formel (IV) seien genannt:

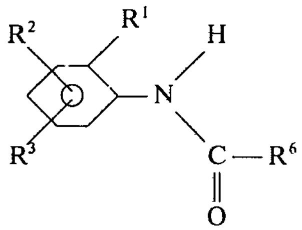

(IV)

| $R^1$ | $R^2$ | $R^3$ | $R^6$ |
|---|---|---|---|
| $CH_3$ | 6-$CH_3$ | H | 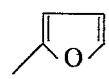 |
| $C_2H_5$ | 6-$CH_3$ | H | 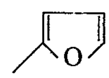 |
| $C_2H_5$ | 6-$C_2H_5$ | H | 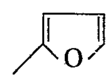 |
| $CH_3$ | 3-$CH_3$ | 6-$CH_3$ | 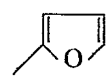 |
| Cl | 6-$CH_3$ | H | 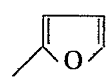 |
| $CH_3$ | 6-$CH_3$ | H | $-CH_2-O-CH_3$ |
| $C_2H_5$ | 6-$CH_3$ | H | $-CH_2-O-CH_3$ |
| $C_2H_5$ | 6-$C_2H_5$ | H | $-CH_2-O-CH_3$ |
| $CH_3$ | 3-$CH_3$ | 6-$CH_3$ | $-CH_2-O-CH_3$ |
| Cl | 6-$CH_3$ | H | $-CH_2-O-CH_3$ |
| $CH_3$ | 6-$CH_3$ | H | $-COOCH_3$ |
| $C_2H_5$ | 6-$CH_3$ | H | $-COOCH_3$ |
| $C_2H_5$ | 6-$C_2H_5$ | H | $-COOCH_3$ |
| $CH_3$ | 3-$CH_3$ | 6-$CH_3$ | $-COOCH_3$ |
| Cl | 6-$CH_3$ | H | $-COOCH_3$ |

Fortsetzung

| $R^1$ | $R^2$ | $R^3$ | $R^6$ |
|---|---|---|---|
| $CH_3$ | 6-$CH_3$ | H | 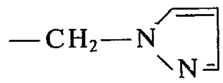 |
| $C_2H_5$ | 6-$CH_3$ | H | 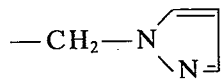 |
| $C_2H_5$ | 6-$C_2H_5$ | H | 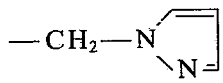 |
| $CH_3$ | 3-$CH_3$ | 6-$CH_3$ | 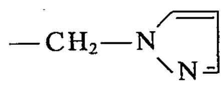 |
| Cl | 6-$CH_3$ | H | 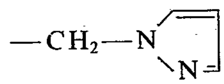 |
| $CH_3$ | 6-$CH_3$ | H | 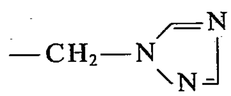 |
| $C_2H_5$ | 6-$CH_3$ | H | 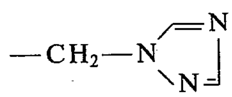 |
| $C_2H_5$ | 6-$C_2H_5$ | H | 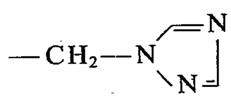 |
| $CH_3$ | 3-$CH_3$ | 6-$CH_3$ | 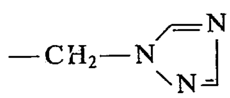 |
| Cl | 6-$CH_3$ | H | 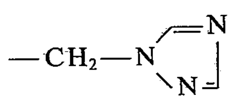 |
| $CH_3$ | 6-$CH_3$ | H | 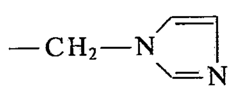 |
| $C_2H_5$ | 6-$CH_3$ | H | 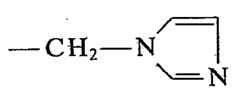 |
| $C_2H_5$ | 6-$C_2H_5$ | H | 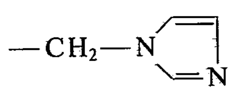 |
| $CH_3$ | 3-$CH_3$ | 6-$CH_3$ | 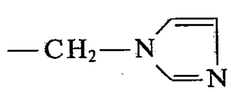 |
| Cl | 6-$CH_3$ | H | 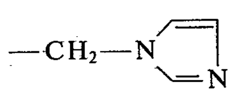 |
| $CH_3$ | 6-$CH_3$ | H | $-CH_2-O-SO_2CH_3$ |
| $C_2H_5$ | 6-$CH_3$ | H | $-CH_2-O-SO_2CH_3$ |

Fortsetzung

| $R^1$ | $R^2$ | $R^3$ | $R^6$ |
|---|---|---|---|
| $C_2H_5$ | 6-$C_2H_5$ | H | $-CH_2-O-SO_2CH_3$ |
| $CH_3$ | 3-$CH_3$ | 6-$CH_3$ | $-CH_2-O-SO_2CH_3$ |
| Cl | 6-$CH_3$ | H | $-CH_2-O-SO_2CH_3$ |
| $CH_3$ | 6-$CH_3$ | H | $-CHCl_2$ |
| $C_2H_5$ | 6-$CH_3$ | H | $-CHCl_2$ |
| $C_2H_5$ | 6-$C_2H_5$ | H | $-CHCl_2$ |
| $CH_3$ | 3-$CH_3$ | H | $-CHCl_2$ |
| Cl | 6-$CH_3$ | H | $-CHCl_2$ |
| $CH_3$ | 6-$CH_3$ | H | $-OC_2H_5$ |
| $C_2H_5$ | 6-$CH_3$ | H | $-OC_2H_5$ |
| $C_2H_5$ | 6-$C_2H_5$ | H | $-OC_2H_5$ |
| $CH_3$ | 3-$CH_3$ | 6-$CH_3$ | $-OC_2H_5$ |
| Cl | 6-$CH_3$ | H | $-OC_2H_5$ |
| $CH_3$ | 6-$CH_3$ | H | 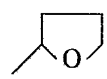 |
| $C_2H_5$ | 6-$CH_3$ | H | 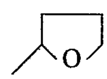 |
| $C_2H_5$ | 6-$C_2H_5$ | H | 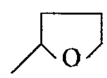 |
| $CH_3$ | 3-$CH_3$ | 6-$CH_3$ | 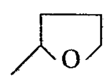 |
| Cl | 6-$CH_3$ | H | 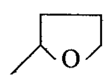 |
| $CH_3$ | 6-$CH_3$ | H | $-CH_2-O-CH_2-O-C_2H_5$ |
| $C_2H_5$ | 6-$CH_3$ | H | $-CH_2-O-CH_2-O-C_2H_5$ |
| $C_2H_5$ | 6-$C_2H_5$ | H | $-CH_2-O-CH_2-O-C_2H_5$ |
| $CH_3$ | 3-$CH_3$ | 6-$CH_3$ | $-CH_2-O-CH_2-O-C_2H_5$ |
| Cl | 6-$CH_3$ | H | $-CH_2-O-CH_2-O-C_2H_5$ |
| $CH_3$ | 6-$CH_3$ | H | $-CH_2-O-$ 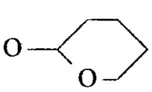 |

Fortsetzung

| $R^1$ | $R^2$ | $R^3$ | $R^6$ |
|---|---|---|---|
| $C_2H_5$ | $6\text{-}CH_3$ | H | $-CH_2-O-$ 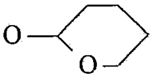 |
| $C_2H_5$ | $6\text{-}C_2H_5$ | H | $-CH_2-O-$ 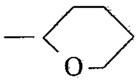 |
| $CH_3$ | $3\text{-}CH_3$ | $6\text{-}CH_3$ | $-CH_2-O-$ 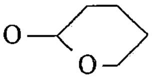 |
| Cl | $6\text{-}CH_3$ | H | $-CH_2-O-$ 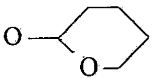 |

Die außerdem für das erfindungsgemäße Verfahren (b) als Ausgangsstoffe zu verwendenden Propargylhalogenide sind durch die Formel (V) allgemein definiert. In dieser Formel stehen $R^4$ und $R^5$ vorzugsweise für diejenigen Reste, die bereits im Zusammenhang mit der Beschreibung der erfindungsgemäßen Stoffe der Formel (I) vorzugsweise für diese Substituenten genannt werden.

Die Propargylhalogenide der Formel (V) sind allgemein bekannte Verbindungen der organischen Chemie.

Die bei der Durchführung des erfindungsgemäßen Verfahrens (c) als Ausgangsstoffe benötigten Halogenacetanilide sind durch die Formel (VI) allgemein definiert. In dieser Formel stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenacetanilide der Formel (VI) sind teilweise bekannt (vergleiche US-Patentschrift 4 001 325). Noch nicht bekannte können nach dem dort beschriebenen Verfahren und entsprechend der Verfahrensvariante (a) erhalten werden, indem man N-Propargyl-aniline der Formel (II) mit Halogenessigsäurehalogeniden umsetzt.

Die außerdem für das erfindungsgemäße Verfahren (c) noch als Ausgangsstoffe zu verwendenden Verbindungen sind durch die Formel (VII) allgemein definiert. In dieser Formel stehen Az und $R^7$ vorzugsweise für diejenigen Reste, die bereits im Zusammenhang mit der Beschreibung der erfindungsgemäßen Stoffe der Formel (I) vorzugsweise genannt wurden. B steht vorzugsweise für Wasserstoff, Natrium und Kalium.

Die Verbindungen der Formel (VII) sind allgemein bekannte Verbindungen der organischen Chemie.

Die bei der Durchführung des erfindungsgemäßen Verfahrens (d) als Ausgangsstoffe benötigten Hydroxyacetanilide sind durch die Formel (VIII) allgemein definiert. In dieser Formel stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Substituenten genannt wurden.

Die Hydroxyacetanilide der Formel (VIII) können auf allgemein bekannte Art und Weise erhalten werden, indem man Acyloxyacetylanilide der Formel

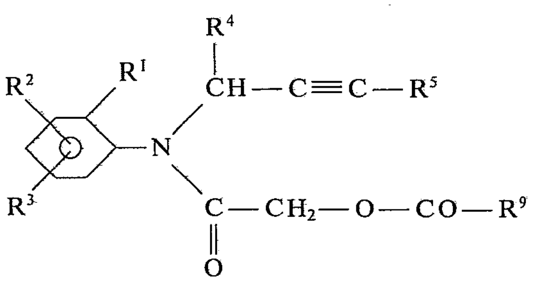

(XI)

in welcher

$R^1$ bis $R^5$ die oben angegebene Bedeutung haben, und

$R^9$ für Alkyl mit 1 bis 4 Kohlenstoffatomen steht,

mit Natron- oder Kalilauge bzw. mit einem Alkalialkoholat eines niederen Alkohols wie z. B. Natriummethylat oder Natriumethylat, bei Temperaturen zwischen 20 und 40°C verseift und nach Ansäuern in üblicher Weise die Verbindungen der Formel (VIII) isoliert.

Die Acyloxyacetylanilide der Formel (XI) können in allgemein üblicher und bekannter Art und Weise erhalten werden, indem man z. B. bei Halogenacetaniliden der Formel (VI) den reaktionsfähigen Substituenten Hal' durch Reaktion mit einer niederen Alkancarbonsäure austauscht, wobei die Säure vorzugsweise in Form ihrer Alkali- oder Erdalkalisalze eingesetzt wird, oder indem man N-Propargyl-aniline der Formel (II) mit entsprechenden Acyloxyacetylhalogeniden umsetzt.

Die außerdem für das erfindungsgemäße Verfahren (d/1) als Ausgangsstoffe zu verwendenden Halogenide sind durch die Formel (IX) definiert. In dieser Formel stehen $R^8$ und Hal' vorzugsweise für die in der Erfindungsdefinition angegebenen Reste.

Die Halogenide der Formel (IX) sind allgemein bekannte Verbindungen der organischen Chemie.

Das für das erfindungsgemäße Verfahren (d/2) außerdem als Ausgangsstoff zu verwendende Dihydropyran ist ebenfalls eine bekannte Verbindung der organischen Chemie.

Die bei der Durchführung des erfindungsgemäßen Verfahrens (e) als Ausgangsstoffe benötigten N-Propargyl-anilide sind durch die Formel (Ia) allgemein definiert. Es handelt sich dabei um erfindungsgemäße Verbindungen, bei denen $R^5$ für Wasserstoff steht.

Als Verdünnungsmittel kommen für die erfindungsgemäße Umsetzung gemäß Verfahrensvariante (a) vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol und Ester, wie Essigester.

Das erfindungsgemäße Verfahren (a) kann gegebenenfalls in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder wie Pyridin ferner anorganische Basen, wie beispielsweise Alkalihydroxide und Alkalicarbonate. Gegebenenfalls kann auch ein Katalysator, wie Dimethylformamid, verwendet werden.

Die Reaktionstemperaturen können bei der Durchführung des erfindungsgemäßen Verfahrens (a) in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens (a) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (b) alle inerten organischen Lösungsmittel in Frage. Hierzu gehören vorzugsweise Ether, wie Diethylether, Dioxan oder Tetrahydrofuran; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Ketone, wie Methylisobutylketon; Nitrile, wie Acetonitril sowie Dimethylformamid oder Dimethylsulfoxid.

Die erfindungsgemäße Umsetzung gemäß Verfahren (b) wird in Gegenwart eines Säurebindemittels durchgeführt. Als solche können alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen −70 und +100°C, vorzugsweise zwischen −20 und +80°C.

Bei der Durchführung des erfindungsgemäßen Verfahrens (b) setzt man vorzugsweise auf 1 Mol Anilid der Formel (IV) 1 bis 1,5 Mol Propargylhalogenid der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wäßrige Natron- oder Kalilauge/Toluol oder Methylenchlorid, unter Zusatz von 0,1—1 Mol eines Phasen-Transfer-Katalysators, wie beispielsweise Ammonium- oder Phosphoniumverbindungen, beispielsweise seien Benzyl-dodecyl-dimethyl-ammoniumchlorid und Triethyl-benzyl-ammoniumchlorid genannt, durchgeführt (vergleiche auch die Herstellungsbeispiele).

Als Verdünnungsmittel kommen für die erfindungsgemäße Umsetzung gemäß Verfahren (c) vorzugsweise inerte organische Lösungsmittel in Frage. Hierzu gehören vorzugsweise die beim Verfahren (a) bereits genannten Lösungsmittel.

Die Umsetzung nach Verfahren (c) kann gegebenenfalls in Gegenwart eines Säurebinders durchgeführt werden. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, Dimethylbenzylamin; oder wie Pyridin und Diazabicyclooctan. Vorzugsweise verwendet man einen Überschuß an Azol.

Die Reaktionstemperaturen können beim Verfahren (c) in einem größeren Bereich variiert werden. Im allgemeinen arbeitet man zwischen etwa 20 bis 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (c) setzt man auf 1 Mol der Verbindungen der Formel (VI) vorzugsweise 1 bis 2 Mol der Verbindungen der Formel (VII) und gegebenenfalls 1 bis 2 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (d) vorzugsweise inerte organische Lösungsmittel in Frage. Hierzu gehören vorzugsweise die beim Verfahren (a) bereits genannten Lösungsmittel.

Die Umsetzung nach Verfahren (d/1) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (d/1) in einem größeren Bereich variiert werden. Im allgemeinen arbeitet man zwischen 20 und 150°C, vorzugsweise bei der Siedetemperatur des Lösungsmittels, beispielsweise zwischen 60 und 100°C.

Bei der Durchführung des erfindungsgemäßen Verfahrens (d/1) setzt man auf 1 Mol der Verbindungen der Formel (VIII), gegebenenfalls nach Zugabe von 1 bis 2 Mol einer starken Base, wie z. B. eines Alkalihydrids, 1 Mol Halogenid der Formel (IX) und gegebenenfalls 1 bis 2 Mol Säurebinder ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Nach einer bevorzugten Ausführungsform des Verfahrens (d/1) wird zweckmäßigerweise so verfahren, daß man von einem Hydroxyacetanilid der Formel (VIII) ausgeht, letzteres in einem geeigneten inerten Lösungsmittel mittels Alkalimetall-hydrid oder -bromid in das Alkalimetall-alkanolat überführt, und letzteres ohne Isolierung sofort mit einem Halogenid der Formel (IX) umsetzt, wobei unter Austritt von Alkalihalogenid die erfindungsgemäßen Verbindungen der Formel (I) in einem Arbeitsgang erhalten werden.

Die Umsetzung nach Verfahren (d/2) kann gegebenenfalls in Gegenwart eines Katalysators durchgeführt werden. Hierzu verwendet man vorzugsweise Chlorwasserstoff (vgl. J. Am. Chem. Soc. 69, 2246 [1947], ibid. 70, 4187 [1948]).

Die Reaktionstemperaturen können beim Verfahren (d/2) in einem größeren Bereich variiert werden. Im allgemeinen arbeitet man zwischen 0 und 100°C, vorzugsweise zwischen 20 und 60°C.

Bei der Durchführung des erfindungsgemäßen Verfahrens (d/2) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (e) Wasser sowie gegenüber Alkalihypohalogenit inerte organische Lösungsmittel in Frage. Hierzu gehören vorzugsweise Alkohole, wie Methanol oder Ethanol; Ether, wie Diethylether, Dioxan oder Tetrahydrofuran, sowie auch Zweiphasengemische, wie z. B. Ether/Wasser. Die Reaktionstemperaturen können beim Verfahren (e) in einem größeren Bereich variiert werden. Im allgemeinen arbeitet man zwischen 0 und 100°C, vorzugsweise zwischen 0 und 40°C.

Bei der Durchführung des erfindungsgemäßen Verfahrens (e) setzt man vorzugsweise auf 1 Mol der Verbindung der Formel (Ia) 1 bis 1,5 Mol Hypohalogenit ein, wobei das Alkalihypohalogenit in-situ aus dem entsprechenden Halogen und dem Alkalihydroxid erzeugt wird. (Vergleiche Houben-Weyl, Bd. V/2a, S. 608—610 [1977]).

Die Isolierung der Endprodukte erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Omycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Omyceten, z. B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht um eine protektive, sondern auch eine kurativ/eradikative Wirkung entfalten. Außerdem besitzen sie systemische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der

Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen,Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Beispiel A

### Phytophthora-Test (Tomaten)/Protektiv

| | |
|---|---|
| Lösungsmittel: | 4,7 Gewichtsteile Aceton |
| Emulgator: | 0,3 Gewichtsteile Alkyl-aryl-polyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20° C und einer relativen Luftfeuchtigkeit von 70% im Gewächshaus. Anschließend werden die Tomatenpflanzen mit einer wäßrigen Sporensuspension

von Phytophthora infestans inokuliert. Die Pflanzen werden in eine Feuchtkammer mit einer 100%igen Luftfeuchtigkeit und einer Temperatur von 18 bis 20° C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

Wirkstoff, Wirkstoffkonzentrationen und Ergebnisse werden ermittelt.

In diesem Test zeigen z. B. folgende Verbindungen eine sehr gute Wirkung, die derjenigen der aus dem Stand der Technik bekannten Verbindungen deutlich überlegen ist:

Verbindungen gemäß Herstellungsbeispielen 1, 2 und 6.

Beispiel B

Phytophthora-Test (Tomaten)/systemisch

| | |
|---|---|
| Lösungsmittel: | 4,7 Gewichtsteile Aceton |
| Dispergiermittel: | 0,3 Gewichtsteile Alkyl-arylpolyglykolether |
| Wasser: | 95,0 Gewichtsteile |

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ccm der Gießflüssigkeit in der angegebenen Wirkstoffkonzentration, bezogen auf 100 ccm Erde, gegossen.

Die so behandelten Pflanzen werden nach der Behandlung mit einer wäßrigen Sporensuspension von Phythohthora infestans inokuliert. Die Pflanzen werden in eine Feuchtkammer mit einer Luftfeuchtigkeit von 100% und einer Temperatur von 18 bis 20° C gebracht. Nach 5 Tagen wird der Befall der Tomtatenpflanzen bestimmt. Die so erhaltenen Boniturwerte werden auf Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt.

In diesem Test zeigen z. B. folgende Verbindungen eine sehr gute Wirkung, die derjenigen der aus dem Stand der Technik bekannten Verbindungen deutlich überlegen ist:

Verbindungen gemäß Herstellungsbeispielen 1, 2 und 6

Herstellungsbeispiele

Beispiel 1

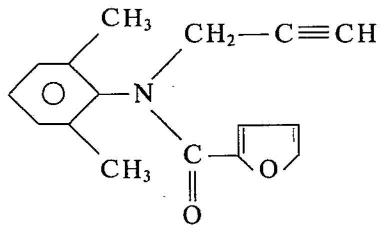

(Verfahren a)

15,9 g (0,1 Mol) 2,6-Dimethyl-N-propargyl-anilin und 8 g (0,1 Mol) Pyridin werden in 100 ml Tetrahydrofuran zum Sieden erhitzt und vorsichtig mit 13 g (0,1 Mol) Furan-2-carbonsäurechlorid versetzt. Man läßt 15 Minuten unter Rückfluß rühren und engt dann die Reaktionsmischung durch Abdestillieren des Lösungsmittels im Vakuum ein. Der Rückstand wird mit Methylenchlorid aufgenommen und mit Wasser gewaschen.

Man trennt die organische Phase ab, trocknet über Natriumsulfat und engt ein. Der Rückstand kristallisiert nach dem Verreiben mit Petrolether. Man erhält 22,5 g (89% der Theorie) 2,6-Dimethyl-N-(2-furoyl)-N-propargyl-anilin vom Schmelzpunkt 109 – 112° C.

Herstellung des Ausgangsproduktes

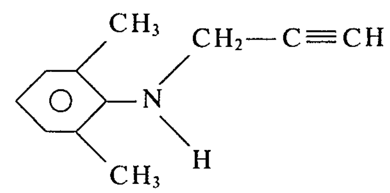

Die Herstellung des 2,6-Dimethyl-N-propargyl-anilins erfolgt durch Umsetzung von 2,6-Dimethyl-anilin mit Propargylbromid entsprechend den Literaturangaben (vgl. US-PS 4 001 325).

(Verfahren b)

21,5 g (0,1 Mol) 2,6-Dimethyl-N-(2-furoyl)-anilin und 0,5 g Triethyl-benzyl-ammoniumchlorid werden in einem Zweiphasengemisch aus 50 ml 50%iger Natronlauge und 250 ml Toluol gelöst und unter heftigem Rühren bei 20 bis 35° C tropfenweise mit 13,1 g (0,11 Mol) Propargylbromid versetzt. Man läßt 2 Stunden bei 20° C rühren, trennt die organische Phase ab, wäscht sie mehrmals mit Wasser, trocknet über Natriumsulfat und engt durch Abdestillieren des Lösungsmittels im Vakuum ein. Der Rückstand kristallisiert nach dem Verreiben mit Petrolether. Man erhält 21 g (83% der Theorie) 2,6-Dimethyl-N-(2-furoyl)-N-propargylanilin vom Schmelzpunkt 110 – 112° C.

Herstellung des Ausgangsproduktes

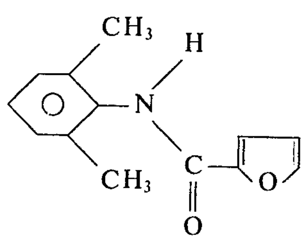

60 g (0,46 Mol) Furan-2-carbonsäurechlorid werden unter Rühren bei 0 bis 10° C zu einer Lösung von 55,7 g (0,46 Mol) 2,6-Dimethyl-anilin und 46,5 g (0,46 Mol) Triethylamin in 500 ml Methylenchlorid getropft. Man läßt zwei Stunden bei 20° C nachrühren und saugt das entstandene Salz ab. Das Filtrat wird mehrmals mit Wasser gewaschen, über Natriumsulfat getrocknet und durch Abdestillieren des Lösungsmittels im Vakuum umkristallisiert. Der Rückstand wird aus Cyclohexan/Toluol umkristallisiert. Man erhält 78,4 kg (79% der Theorie) 2,6-Dimethyl-N-(2-furoyl)-anilin vom Schmelzpunkt 119 – 122° C.

Beispiel 2

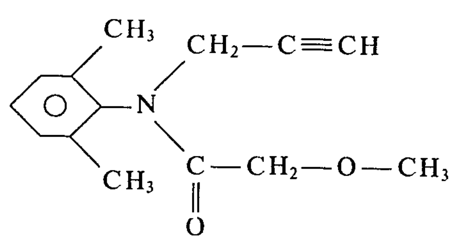

(Verfahren b)

30 g (0,155 Mol) 2,6-Dimethyl-N-methoxyacetyl-anilin und 0,3 g Triethyl-benzyl-ammoniumchlorid werden in einem Zweiphasensystemgemisch aus 100 ml 50%iger Natronlauge und 250 ml Toluol gelöst und unter heftigem Rühren mit 19 g (0,016 Mol) Propargylbromid versetzt. Man läßt 4 Stunden rühren, trennt die Toluolphase ab, wäscht sie mehrfach mit Wasser, trocknet über Natriumsulfat und engt durch Abdestillieren des Lösungsmittels im Wasserstrahlvakuum ein. Der Rückstand wird im Hochvakuum destilliert. Man erhält 26,5 g (74% der Theorie) 2,6-Dimethyl-N-methoxyacetyl-N-propargyl-anilin vom Siedepunkt 140° C/0,5 Torr und vom Schmelzpunkt 49 – 51° C.

Herstellung des Ausgangsproduktes

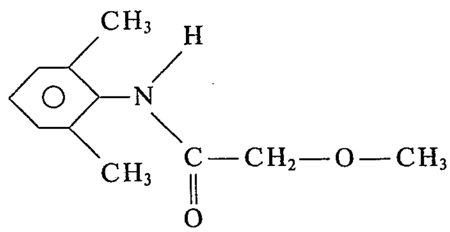

55 g (0,5 Mol) Methoxyacetylchlorid werden bei 5 bis 15°C unter Rühren und Kühlen in eine Lösung von 61 g (0,5 Mol) 2,6-Dimethylanilin und 50,5 g (0,5 Mol) Triethylamin in 250 ml Toluol getropft. Man läßt 2 Stunden bei 20°C nachrühren, filtriert, engt ein und nimmt den Rückstand mit Wasser auf. Man extrahiert mit Methylenchlorid, trocknet über Natriumsulfat und engt durch Abdestillieren des Lösungsmittels ein. Der Rückstand wird im Hochvakuum destilliert. Man erhält 55 g (57% der Theorie) 2,6-Dimethyl-N-methoxyacetyl-anilin vom Siedepunkt 122–132°C/0,1 Torr und vom Schmelzpunkt 61–63°C.

Beispiel 3

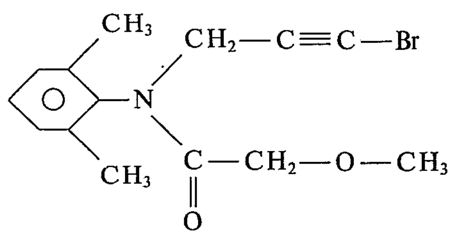

(Verfahren e)

10 g (0,063 Mol) Brom werden bei −5°C in eine Lösung von 22,4 kg (0,4 Mol) Kaliumhydroxid in 100 ml Wasser getropft. Man läßt auf 0°C erwärmen und tropft unter starkem Rühren bei dieser Temperatur eine Lösung von 16,6 g (0,05 Mol) 2,6-Dimethyl-N-methoxyacetyl-N-propargyl-anilin (Beispiel 2) in 150 ml Ester zu. Nach einer Stunde Rühren bei 20°C wird die Etherphase abgetrennt, nochmals mit Wasser gewaschen, über Natriumsulfat getrocknet und durch Abdestillieren des Ethers eingeengt. Der Rückstand kristallisiert nach Verreiben mit Petrolether. Man erhält 10,1 g (65% der Theorie) 2,6-Dimethyl-N-(3-Brom-propargyl)-N-methoxyacetyl-anilin vom Schmelzpunkt 53–54°C.

Beispiel 4

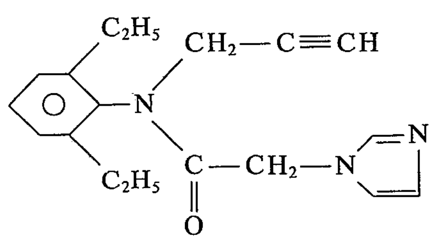

(Verfahren c)

Ein Gemisch aus 13,2 g (0,05 Mol) 2,6-Diethyl-N-propargyl-chloracetanilid und 13,6 g (0,2 Mol) Imidazol wird als Schmelze bei 120°C 30 Minuten gerührt. Danach wird das Reaktionsgemisch auf Wasser gegeben, der Niederschlag abgesaugt und gründlich mit Wasser gewaschen. Nach dem Umkristallisieren aus Diisopropylether/Essigester erhält man 10 g (68% der Theorie 2,6-Diethyl-N-(imidazol-1-yl-acetyl)-N-propargyl-anilin vom Schmelzpunkt 129–131°C.

Herstellung des Ausgangsproduktes

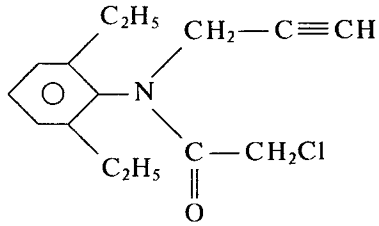

Die Herstellung erfolgt durch Umsetzung von 2,6-Diethylanilin mit Propargylbromid in Gegenwart von Kaliumcarbonat sowie weitere Umsetzung des erhaltenen 2,6-Diethyl-propargylanilins mit Chloressigsäurechlorid oder -anhydrid entsprechend den Literaturangaben (vgl. US-PS 4 001 325).

In analoger Weise werden die Verbindungen der allgemeinen Formel:

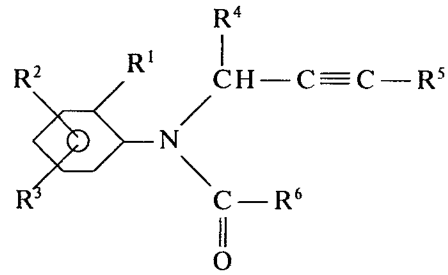

(I)

erhalten:

| Bsp. Nr. | $R^1$ | $R^2$ | $R^3$ | $R^4$ | $R^5$ | $R^6$ | Schmelzpunkt (°C) |
|---|---|---|---|---|---|---|---|
| 5 | $CH_3$ | 6-$C_2H_5$ | H | H | H | —$CH_2$— 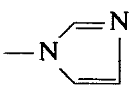 | 129 – 31 |
| 6 | $CH_3$ | 6-$CH_3$ | H | H | $CH_3$ | 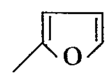 | 110 – 12 |
| 7 | $CH_3$ | 6-$CH_3$ | H | H | H | —$CH_2$— 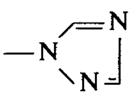 | 120 – 21 |
| 8 | $CH_3$ | 6-$CH_3$ | H | H | H | —$CH_2$— 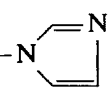 | 137 – 40 |
| 9 | $CH_3$ | 6-$C_2H_5$ | H | H | H | 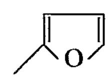 | 88 |
| 10 | $CH_3$ | 6-$CH_3$ | H | H | Br | 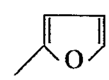 | 138 – 40 |
| 11 | $CH_3$ | 6-$CH_3$ | H | H | $CH_3$ | —$CH_2$—O—$CH_3$ | $n_D^{20}$ : 1,5362<br>$Kp_{0,1}$ : 125°C |
| 12 | $C_2H_5$ | 6-$C_2H_5$ | H | H | H | —$CH_2$—O—$CH_3$ | 74 |
| 13 | $CH_3$ | 6-$CH_3$ | H | H | H | —$CHCl_2$ | 114 – 15 |

Fortsetzung

| Bsp. Nr. | $R^1$ | $R^2$ | $R^3$ | $R^4$ | $R^5$ | $R^6$ | Schmelzpunkt (°C) |
|---|---|---|---|---|---|---|---|
| 14 | $CH_3$ | 6-$CH_3$ | H | H | H | 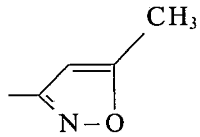 | 98 – 99 |
| 15 | Cl | 6-$CH_3$ | H | H | H | —$CH_2$—O—$CH_3$ | 80 |
| 16 | $CH_3$ | 6-$CH_3$ | H | H | H | —$CH_2$—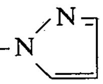 | 93 – 94 |
| 17 | $CH_3$ | 6-$CH_3$ | H | $CH_3$ | H | —$CH_2OCH_3$ | Öl |
| 18 | $CH_3$ | 6-$CH_3$ | H | $CH_3$ | Br | —$CH_2OCH_3$ | Öl |
| 19 | $CH_3$ | 6-$CH_3$ | H | $CH_3$ | H | —$CH_2$—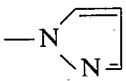 | 84 – 86 |
| 20 | $CH_3$ | 6-$CH_3$ | H | $CH_3$ | H | —$CH_2$—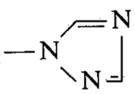 | 129 – 30 |
| 21 | $CH_3$ | 6-$CH_3$ | H | $CH_3$ | H | 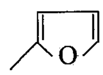 | 87 – 89 |
| 22 | $CH_3$ | 6-$CH_3$ | H | $CH_3$ | H | —$COOCH_3$ | 63 – 65 |
| 23 | $CH_3$ | 6-$CH_3$ | H | $CH_3$ | H | —$CH_2$—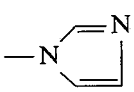 | 235 – 37 (xHCl) |
| 24 | $CH_3$ | 6-$CH_3$ | H | $CH_3$ | H | —$CH_2$—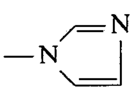 | 273 – 74 |
| 25 | $CH_3$ | 6-$CH_3$ | H | $CH_3$ | H | —$CH_2OSO_2CH_3$ | Öl |
| 26 | $CH_3$ | 6-$CH_3$ | H | $CH_3$ | H | —$CH_2O$—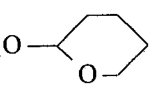 | Öl |
| 27 | $CH_3$ | 6-$CH_3$ | H | $CH_3$ | H | —$CHCl_2$ | 81 – 82 |
| 28 | $CH_3$ | 6-$CH_3$ | H | $CH_3$ | H | —$CH_2SCH_3$ | $n_D^{21}$ : 1,5470 |
| 29 | $CH_3$ | 6-$CH_3$ | H | $CH_3$ | H | —$CH_2$—SCN | 69 – 70 |
| 30 | $CH_3$ | 6-$CH_3$ | H | $CH_3$ | H | —$C(CH_3){=}CH_2$ | $n_D^{28}$ : 1,5272 |
| 31 | $CH_3$ | 6-$CH_3$ | H | $CH_3$ | H | —$CH_2CH_2OCH_3$ | $n_D^{23}$ : 1,5150 |
| 32 | $CH_3$ | 6-$CH_3$ | H | $CH_3$ | H | —$CH{=}CH$—$CH_3$ | $n_D^{22,5}$ : 1,5377 |
| 33 | $CH_3$ | 6-$CH_3$ | H | $CH_3$ | H | —$C_3H_7$-n | $n_D^{23}$ : 1,5102 |
| 34 | $CH_3$ | 6-$CH_3$ | H | $CH_3$ | H | —$CH_2OC_3H_7$ | $n_D^{22,5}$ : 1,5088 |

Fortsetzung

| Bsp. Nr. | $R^1$ | $R^2$ | $R^3$ | $R^4$ | $R^5$ | $R^6$ | Schmelzpunkt (°C) |
|---|---|---|---|---|---|---|---|
| 35 | $CH_3$ | 6-$CH_3$ | H | $CH_3$ | H | $-C_3H_7$-i | 70 – 72 |
| 36 | $CH_3$ | 6-$CH_3$ | H | H | H | $-COOCH_3$ | 61 – 62 |
| 37 | $C_2H_5$ | 6-$C_2H_5$ | H | H | H | 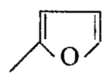 | 96 – 97 |
| 38 | $CH_3$ | 6-$CH_3$ | H | H | H | $-CH_2OC_2H_5$ | $n_D^{20}$ : 1,5290 |
| 39 | $C_2H_5$ | 6-$C_2H_5$ | H | H | Br | $-CH_2OCH_3$ | 55 – 57 |
| 40 | $CH_3$ | 6-$CH_3$ | H | H | H | $-CH_2OCH_2C{\equiv}CH$ | 56 – 60 |
| 41 | $CH_3$ | 6-$CH_3$ | H | H | H | $-CH_2SCH_3$ | $n_D^{20}$ : 1,5637 |

**Patentansprüche**

1. Substituierte N-Propargyl-aniline der allgemeinen Formel

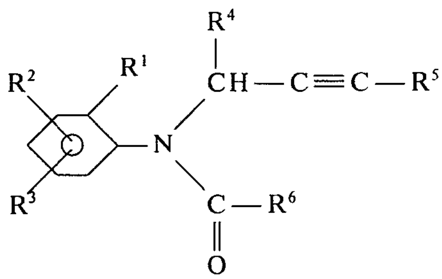

(I)

in welcher
$R^1$, $R^2$, $R^3$, $R^4$ und $R^5$ für Wasserstoff und
Alkyl mit 1 bis 4 Kohlenstoffatomen stehen, und darüber hinaus noch
$R^1$ zusätzlich für Halogen und
$R^5$ zusätzlich für gegebenenfalls durch Halogen, Alkyl mit 1 bis 4 Kohlenstoffatomen und Nitro substituiertes Phenyl und für Halogen steht,
$R^6$ für Tetrahydrofuryl, Thiophenyl, Tetrahydrothiophenyl, für gegebenenfalls durch Methyl oder Ethyl substituiertes Isooxazolyl, sowie für gegebenenfalls durch Cyano oder Thiocyano substituiertes Alkyl mit 1 bis 4 und Alkenyl und Alkinyl mit jeweils 2 bis 4 Kohlenstoffatomen steht, sowie für die Gruppierungen

$-CH_2-Az$, $-CH_2-OR^8$, $-CH_2-SR^7$, $-OR^7$, $-CH_2-OSO_2R^8$,

$-COOR^7$ und $-CH_2-O-$ 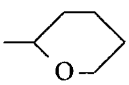

steht, wobei

$R^7$ und $R^8$ für durch Halogen, Cyano und Thiocyano substituiertes Alkyl mit 1 bis 4 Kohlenstoffatomen und für gegebenenfalls durch die genannten Alkyl-Substituenten substituiertes Alkenyl und Alkinyl mit jeweils 2 bis 4 Kohlenstoffatomen und für Alkoxyalkyl mit 1 bis 4 Kohlenstoffatomen in jedem Alkylteil steht, und darüber hinaus noch
$R^7$ zusätzlich für unsubstituiertes Alkyl mit 1 bis 4 Kohlenstoffatomen stehen kann, und
Az für Pyrazol-1-yl, 1,2,4-Triazol-1-yl und Imidazol-1-yl steht.

2. Verfahren zur Herstellung von substituierten N-Propargylanilinen der allgemeinen Formel

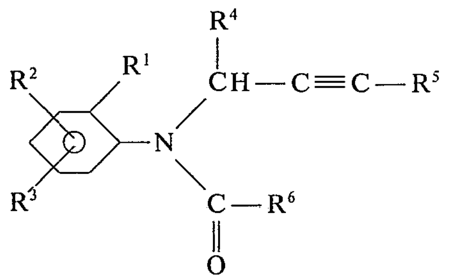

(I)

in welcher
$R^1$, $R^2$, $R^3$, $R^4$ und $R^5$ für Wasserstoff und
Alkyl mit 1 bis 4 Kohlenstoffatomen stehen, und darüber hinaus noch
$R^1$ zusätzlich für Halogen und
$R^5$ zusätzlich für gegebenenfalls durch Halogen, Alkyl, mit 1 bis 4 Kohlenstoffatomen und Nitro substituiertes Phenyl und für Halogen steht,
$R^6$ für Tetrahydrofuryl, Thiophenyl, Tetrahydrothiophenyl, für gegebenenfalls durch Methyl oder Ethyl substituiertes Isoxazolyl, sowie für gegebenenfalls durch Cyano oder Thiocyano substituiertes Alkyl mit 1 bis 4 und Alkenyl und Alkinyl mit jeweils 2 bis 4 Kohlenstoffatomen steht, sowie die Gruppierungen

$-CH_2-Az$, $-CH_2-OR^8$, $-CH_2-SR^7$, $-OR^7$, $-CH_2-OSO_2R^8$,

$-COOR^7$ und $-CH_2-O-$ 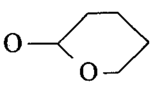

steht, wobei
$R^7$ und $R^8$ für durch Halogen, Cyano und Thiocyano substituiertes Alkyl mit 1 bis 4 Kohlenstoffatomen und für gegebenenfalls durch die genannten Alkyl-Substituenten substituiertes Alkenyl und Alkinyl mit jeweils 2 bis 4 Kohlenstoffatomen und für Alkoxyalkyl mit 1 bis 4 Kohlenstoffatomen in jedem Alkylteil steht, und darüber hinaus noch
$R^7$ zusätzlich für unsubstituiertes Alkyl mit 1 bis 4 Kohlenstoffatomen stehen kann, und
Az für Pyrazol-1-yl, 1,2,4-Triazol-1-yl und Imidazol-1-yl steht,

dadurch gekennzeichnet, daß man

a) N-Propargyl-aniline der Formel

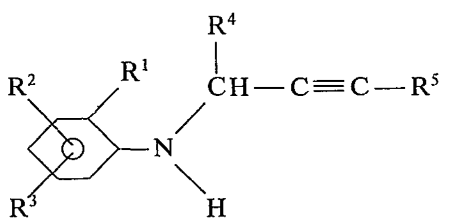

(II)

in welcher
$R^1$ bis $R^5$ die oben angegebene Bedeutung haben,
mit Säurechloriden oder -bromiden bzw. -anhydriden der Formeln

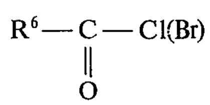

(IIIa)

bzw.

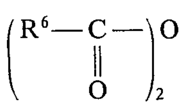

(IIIb)

in welchen
$R^6$ die oben angegebene Bedeutung hat, in Gegenwart eines Verdünnungsmittels und gegebenenfalls in Gegenwart eines Säurebindemittels umsetzt, oder

b) Anilide der Formel

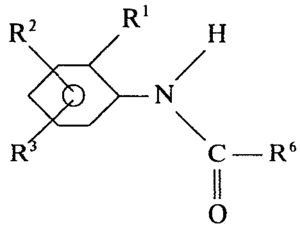

(IV)

in welcher
$R^1$ bis $R^3$ und $R^6$ die oben angegebene Bedeutung haben,
mit Propargylhalogeniden der Formel

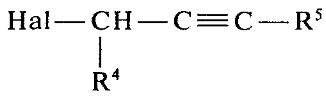

(V)

in welcher
$R^4$ und $R^5$ die oben angegebene Bedeutung haben, und
Hal für Chlor oder Brom steht,

in Gegenwart eines Säurebinders und gegebenenfalls in Gegenwart eines organischen Verdünnungsmittels oder in einem wäßrig-organischen Zweiphasensystem in Gegenwart eines Phasentransferkatalysators umsetzt; oder einzelne Verbindungen der Formel (I), indem man

c) Halogenacetanilide der Formel

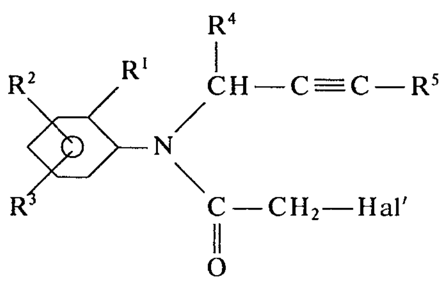

(VI)

in welcher
$R^1$ bis $R^5$ die oben angegebene Bedeutung haben, und
Hal' für Chlor, Brom oder Jod steht,
mit Verbindungen der Formel

$$B-X \qquad \text{(VII)}$$

in welcher
X für Az und die Gruppierung $-OR^8$ oder $-SR^7$ steht, und
B für Wasserstoff oder ein Alkalimetall steht, und
$R^7$ und $R^8$ die oben angegebene Bedeutung haben,

gegebenenfalls in Gegenwart eines Verdünnungsmittels und gegebenenfalls in Gegenwart eines Säurebinders umsetzt, oder

d) Hydroxyacetanilide der Formel

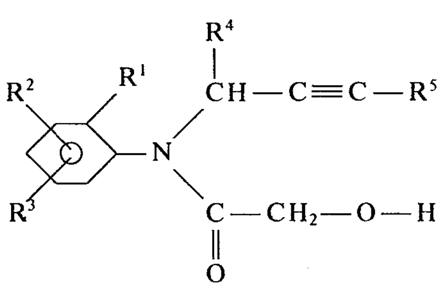

(VIII)

in welcher
$R^1$ bis $R^5$ die oben angegebene Bedeutung haben,

(1) gegebenenfalls nach Aktivierung mittels Alkalimetall mit Halogeniden der Formel

$$Hal'—R^9 \quad (IX)$$

in welcher
Hal' die oben angegebene Bedeutung hat, und
$R^9$ für den Rest $R^8$ sowie die Gruppe $-SO_2R^8$ steht, wobei
$R^8$ die oben angegebene Bedeutung hat,

in Gegenwart eines Verdünnungsmittels und gegebenenfalls in Gegenwart eines Säurebinders umsetzt, oder

(2) mit Dihydropyran der Formel

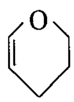

(X)

in Gegenwart eines Verdünnungsmittels und gegebenenfalls in Gegenwart eines Katalysators umsetzt, oder

e) erfindungsgemäße N-Propargyl-anilide der Formel

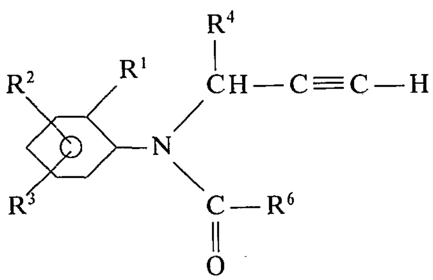

(Ia)

in welcher
$R^1$ bis $R^4$ und $R^6$ die oben angegebene Bedeutung haben,

in an sich bekannter Art und Weise mit wäßrigen Alkalihypohalogenit-Lösungen umsetzt.

3. Fungizide Mittel, gekennzeichnet durch einen Gehalt an mindestens einem substituierten N-Propargylanilin gemäß Ansprüchen 1 und 2.

4. Verwendung von substituierten N-Propargyl-anilinen gemäß Ansprüchen 1 und 2 zur Bekämpfung von Pilzen.

**Claims**

1. Substituted N-propargyl-anilines of the general formula

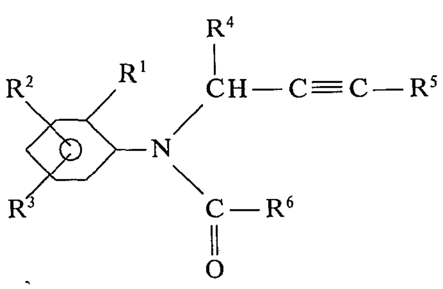

(I)

in which
$R^1$, $R^2$, $R^3$, $R^4$ and $R^5$ represent hydrogen and
alkyl with 1 to 4 carbon atoms, and furthermore
$R^1$ additionally represents halogen and
$R^5$ additionally represents phenyl which is optionally substituted by halogen, alkyl with 1 to 4 carbon

atoms and nitro, and halogen,

$R^6$ represents tetrahydrofuryl, thiophenyl, tetrahydrothiophenyl, optionally methyl- or ethyl-substituted isoxazolyl, and optionally cyano- or thiocyano-substituted alkyl with 1 to 4 and alkenyl and alkinyl each with 2 to 4 carbon atoms, and represents the groupings

$$—CH_2—Az, \quad —CH_2—OR^8, \quad —CH_2—SR^7, \quad —OR^7, \quad —CH_2—OSO_2R^8,$$

$—COOR^7$ and $—CH_2—O—$ 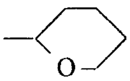

wherein

$R^7$ and $R^8$ represent halogen-, cyano and thiocyano-substituted alkyl with 1 to 4 carbon atoms and alkenyl and alkinyl, with in each case 2 to 4 carbon atoms, which are optionally substituted by the indicated alkyl substituents and represents alkoxyalkyl with 1 to 4 carbon atoms in each alkyl part, and furthermore

$R^7$ can additionally represent unsubstituted alkyl with 1 to 4 carbon atoms, and

Az represents pyrazol-1-yl) 1,2,4-triazol-1-yl and imidazol-1-yl.

2. Process for the preparation of substituted N-propargyl-anilines of the general formula

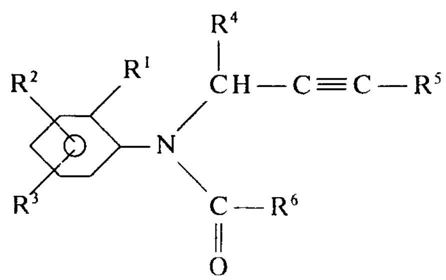

(I)

in which

$R^1$, $R^2$, $R^3$, $R^4$ and $R^5$ represent hydrogen and alkyl with 1 to 4 carbon atoms, and furthermore

$R^1$ additionally represents halogen and

$R^5$ additionally represents phenyl with which is optionally substituted by halogen, alkyl with 1 to 4 carbon atoms and nitro, and halogen,

$R^6$ represents tetrahydrofuryl, thiophenyl, tetrahydrothiophenyl, optionally methyl- oder ethyl-substituted isoxazolyl, and optionally cyano- or thiocyano-substituted alkyl with 1 to 4 and alkenyl and alkinyl each with 2 to 4 carbon atoms, and represents the groupings

$$—CH_2—Az, \quad —CH_2—OR^8, \quad —CH_2—SR^7, \quad —OR^7, \quad —CH_2—OSO_2R^8,$$

$—COOR^7$ and $—CH_2—O—$ 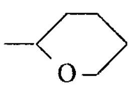

wherein

$R^7$ and $R^8$ represent halogen-, cyano- and thiocyano-substituted alkyl with 1 to 4 carbon atoms and alkenyl and alkinyl, with in each case 2 to 4 carbon atoms, which are optionally substituted by the indicated alkyl substituents and represents alkoxyalkyl with 1 to 4 carbon atoms in each alkyl part, and furthermore

$R^7$ can additionally represent unsubstituted alkyl with 1 to 4 carbon atoms, and

Az represents pyrazol-1-yl, 1,2,4-triazol-1-yl and imidazol-1-yl,

characterized in that

a) N-propargyl-anilines of the formula

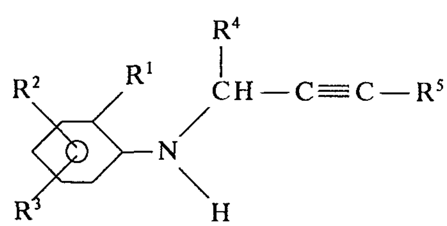

(II)

in which
$R^1$ to $R^5$ have the abovementioned meaning,
are reacted with acid chlorides or acid bromides or acid anhydrides of the formulae

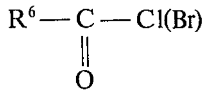
(IIIa)

or

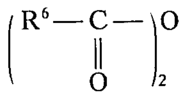
(IIIb)

in which
$R^6$ has the abovementioned meaning in the presence of a diluent and, if appropriate, in the presence of an acid-binding agent, or

b) anilides of the formula

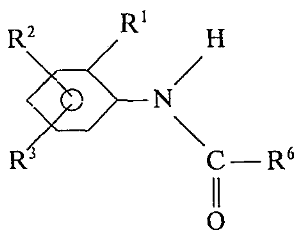
(IV)

in which
$R^1$ to $R^3$ and $R^6$ have the abovementioned meaning are reacted with propargyl halides of the formula

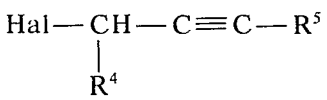
(V)

in which
$R^4$ and $R^5$ have the abovementioned meaning and
Hal represents chlorine or bromine,
in the presence of an acid-binding agent and, if appropriate, in the presence of an organic diluent, or in an aqueous-organic two-phase system in the presence of a phase transfer catalyst; or, in the case of some compounds of the formula (I) in that

c) halogenacetanilides of the formula

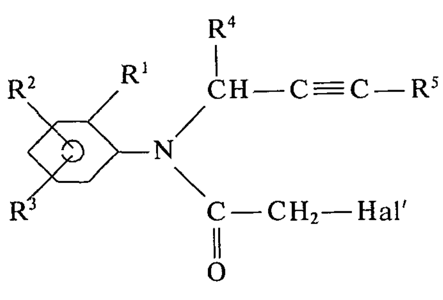
(VI)

in which
$R^1$ to $R^5$ have the abovementioned meaning and
Hal' represents chlorine, bromine or iodine
are reacted with compounds of the formula

$$B—X \quad (VII)$$

in which
X represents Az and the $-OR^8$ or $-SR^7$ group and
B represents hydrogen or an alkali metal and

$R^7$ and $R^8$ have the abovementioned meaning,

if appropriate in the presence of a diluent and, if appropriate, in the presence of an acid-binding agent, or

d) hydroxyacetanilides of the formula

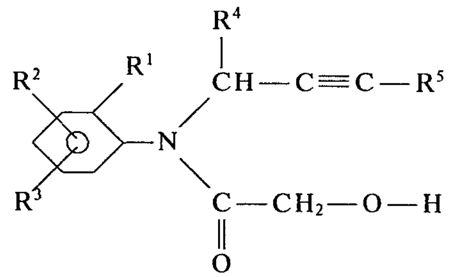

(VIII)

in which
$R^1$ to $R^5$ habe the abovementioned meaning

(1) are reacted, if appropriate after activation by means of an alkali metal, with halides of the formula

$$Hal'-R^9 \quad (IX)$$

in which
Hal' has the abovementioned meaning and
$R^9$ represents the radical $R^8$ or the group $-SO_2R^8$, wherein
$R^8$ has the abovementioned meaning,

in the presence of a diluent and, if appropriate, in the presence of an acid-binding agent, or

(2) are reacted with dihydropyrane of the formula

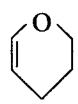

(X)

in the presence of a diluent and, if appropriate, in the presence of a catalyst, or

e) N-propargyl-anilides according to the invention, of the formula

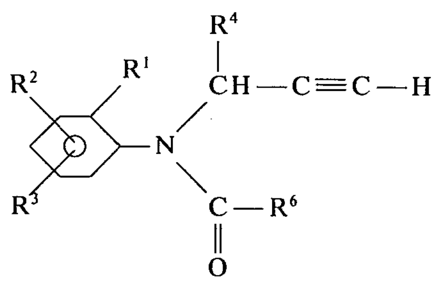

(Ia)

in which
$R^1$ to $R^4$ and $R^6$ have the abovementioned meaning are reacted, in a manner which is in itself known, with aqueous alkali metal hypohalite solutions.

3. Fungicidal agent, characterized in that it contains at least one substituted N-propargyl-aniline according to Claims 1 and 2.

4. Use of substituted N-propargyl-anilines according to Claims 1 and 2 for combating fungi.

**Revendications**

1. Des N-propargyl-anilines substituées de formule générale:

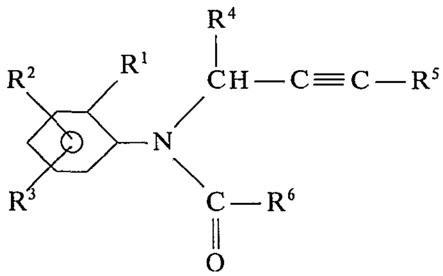

(I)

dans laquelle

$R^1$, $R^2$, $R^3$, $R^4$ et $R^5$ représentent de l'hydrogène et un reste alkyle ayant 1 à 4 atoms de carbone et en outre,

$R^1$ représente également un halogène et

$R^5$ représente également un reste phényle éventuellement substitué par un halogène, par un radical alkyle ayant 1 à 4 atomes de carbone et par le radical nitro, et un halogène,

$R^6$ représente le groupe tétrahydrofuryle, thiophényle, tétrahydrothiophényle, un reste isoxazolyle éventuellement substitué par un radical méthyle ou éthyle ainsi qu'un reste alkyle ayant 1 à 4 et un reste alcényle et alcynyle ayant chacun 2 à 4 atomes de carbone éventuellement substitués par un radical cyano ou thiocyano, ainsi que les groupements

$-CH_2-Az$, $-CH_2-OR^8$, $-CH_2-SR^7$, $-OR^7$, $-CH_2-OSO_2R^8$,

$-COOR^7$ et $-CH_2-O-$ 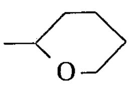

où

$R^7$ et $R^8$ représentent un reste alkyle ayant 1 à 4 atomes de carbone substitué par un halogène, un radical cyano et thiocyano et un reste alcényle et alcynyle ayant chacun 2 à 4 atomes de carbone éventuellement substitués par les substituants alkyle mentionnés et un reste alkoxyalkyle ayant 1 à 4 atomes de carbone dans chaque partie alkylique, et en outre

$R^7$ peut représenter également un reste alkyle non substitué ayant 1 à 4 atomes de carbone et

Az est un reste pyrazol-1-yle, 1,2,4-triazol-1-yl et imidazol-1-yle.

2. Procédé de production de N-propargylanilines de formule générale:

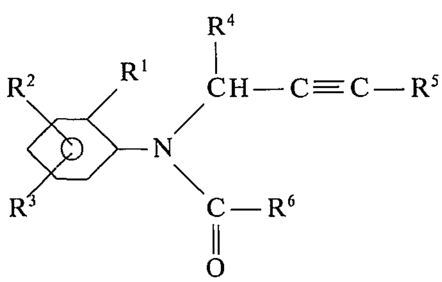

(I)

dans laquelle

$R^1$, $R^2$, $R^3$, $R^4$ et $R^5$ représentent de l'hydrogène et un reste alkyle ayant 1 à 4 atomes de carbone et en outre,

$R^1$ représente également un halogène et

$R^5$ représente également un reste phényle éventuellement substitué par un halogène, par un radical alkyle ayant 1 à 4 atomes de carbone et par un radical nitro, et un halogène,

$R^6$ représente le groupe tétrahydrofuryle, thiophényle, tétrahydrothiophényle, un reste isoxazolyle éventuellement substitué par un radical méthyle ou éthyle ainsi qu'un reste alkyle ayant 1 à 4 et un reste alcényle et alcynyle ayant chaun 2 à 4 atomes de carbone éventuellement substitués par un radical cyano ou thiocyano, ainsi que les groupements

$-CH_2-Az$, $-CH_2-OR^8$, $-CH_2-SR^7$, $-OR^7$, $-CH_2-OSO_2R^8$,

$-COOR^7$ et 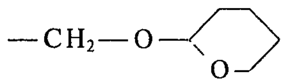

où

$R^7$ et $R^8$ représent un reste alkyle ayant 1 à 4 atomes de carbone substitué par un halogène, un radical cyano et thiocyano et un reste alcényle et alcynyle ayant chaun 2 à 4 atomes de carbone éventuellement substitués par les substituants alkyle mentionnés et un reste alkoxyalkyle ayant 1 à 4 atomes de carbone dans chaque partie alkylique, et en outre

$R^7$ peut représenter également un reste alkyle non substitué ayant 1 à 4 atomes de carbone et

Az est un reste pyrazol-1-yle, 1,2,4-triazol-1-yle et imidazol-1-yle,

caractérisé en ce que

a) On fait réagir des N-propargyl-anilines de formule:

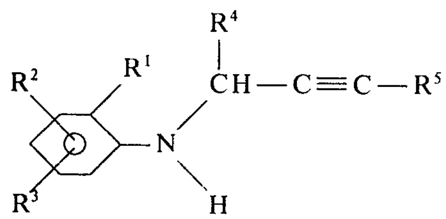

(II)

dans laquelle

$R^1$ à $R^5$ ont la définition indiquée ci-dessus, avec les chlorures ou bromures ou des anhydrides d'acides de formules:

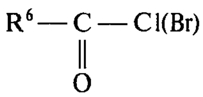

(IIIa)

et respectivement

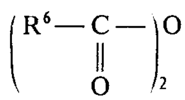

(IIIb)

où

$R^6$ a la définition indiquée ci-dessus, en présence d'un diluant et le cas échéant en présence d'un accepteur d'acide, où

b) On fait réagir des anilides de formule:

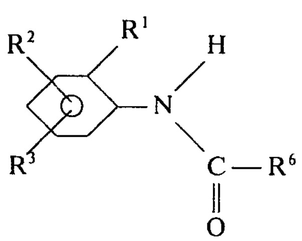

(IV)

dans laquelle

$R^1$ à $R^3$ et $R^6$ ont la définition indiquée ci-dessus, avec des halogénures de propargyle de formule:

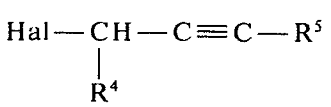

(V)

dans laquelle

$R^4$ et $R^5$ ont la définition indiquée ci-dessus, et

Hal est du chlore ou du brome,

en présence d'un accepteur d'acide et le cas échéant en présence d'un diluant organique ou dans

un système hydroorganique de deux phases en présence d'un catalyseur de transfert de phase; ou des composés individuels de formule (I), par le fait que,

c) On fait réagir des halogénacétanilides de formule:

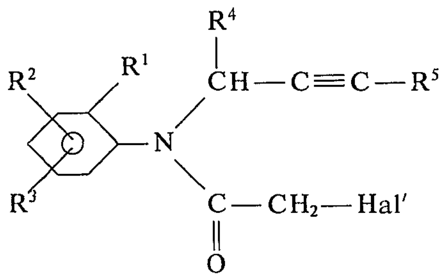

(VI)

dans laquelle
$R^1$ à $R^5$ ont la définition indiquées ci-dessus, et
Hal' représente du chlore, du brome ou de l'iode, avec des composés de formule:

$$B—X \quad (VII)$$

dans laquelle
X représente Az et le groupement $-OR^8$ ou $-SR^7$, et
B est de l'hydrogène ou un métal alcalin, et
$R^7$ et $R^8$ ont la définition indiquée ci-dessus, éventuellement en présence d'un diluant et le cas échéant en présence d'un accepteur d'acide, ou

d) On fait réagir des hydroxyacétanilides de formule:

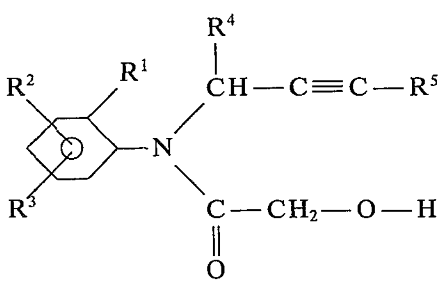

(VIII)

dans laquelle
$R^1$ à $R^5$ ont la définition indiquée ci-dessus,

(1) éventuellement après activation au moyen d'un métal alcalin avec des halogénures de formule:

$$Hal'—R^9 \quad (IX)$$

dans laquelle
Hal' a la définition indiquée ci-dessus, et
$R^9$ représente le reste $R^8$ ainsi que le groupe $-SO_2R^8$,
$R^8$ ayant la définition indiquée ci-dessus,

en présence d'un diluant et le cas échéant en présence d'un accepteur d'acide, ou

(2) avec le dihydropyrane de formule:

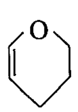

(X)

en présence d'un diluant et en la présence éventuelle d'un catalyseur, ou

e) on fait réagir des N-propargyl-anilides de formule:

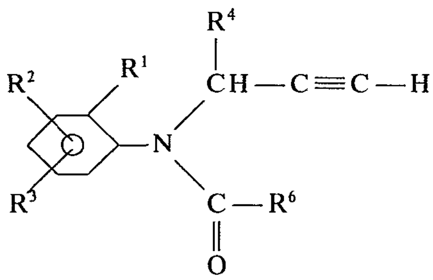

(Ia)

dans laquelle
$R^1$ à $R^4$ et $R^6$ ont la définition indiquée ci-dessus,
d'une manière connue avec des solutions aqueuses d'hypohalogenites alcalins.

3. Compositions fongicides, caractérisées par une teneur en au moins une N-propargyl-aniline substituée suivant les revendications 1 et 2.

4. Utilisation de N-propargyl-anilines substituées suivant les revendications 1 et 2 pour la lutte contre des champignons.